(19) Europäisches Patentamt
European Patent Office
Office européen des brevets

(11) EP 1 164 138 A1

(12) EUROPEAN PATENT APPLICATION

(43) Date of publication:
19.12.2001 Bulletin 2001/51

(21) Application number: 01121776.7

(22) Date of filing: 24.01.1997

(51) Int Cl.7: C07F 15/00, C07D 213/06, C07D 213/22, C07H 19/067, C07H 19/073, C07H 19/167, C07H 19/173

(84) Designated Contracting States:
AT BE CH DE DK ES FI FR GB IE IT LI NL SE

(30) Priority: 26.01.1996 GB 9601603

(62) Document number(s) of the earlier application(s) in accordance with Art. 76 EPC:
97901187.1 / 0 885 233

(71) Applicant: ISIS INNOVATION LIMITED
Summertown, Oxford OX2 7SG (GB)

(72) Inventor: Lowe, Gordon
Oxford OX1 3QY (GB)

(74) Representative:
Ellis-Jones, Patrick George Armine et al
J.A. KEMP & CO. 14 South Square Gray's Inn
London WC1R 5JJ (GB)

Remarks:
This application was filed on 19 - 09 - 2001 as a divisional application to the application mentioned under INID code 62.

# (54) Terpyridine-platinum (II) complexes

(57) A new class of 2,2':6',2"-terpyridine-platinum (II) and substituted 2,2':6',2"-terpyridine-platinum (II) complexes in which an N- or O- or halo nucleophile is the fourth ligand to platinum. The compounds are potent intercalators of DNA. Some have antitumour activity. Some have anti-parasitic activity. A new method of preparing the complexes involves reacting a Pt complex of 1,5-cyclooctadiene with a 2,2':6',2"-terpyridine.

EP 1 164 138 A1

## Description

### Background and Summary

This invention relates to a new class of 2,2':6',2"-terpyridine-platinum (II) and substituted 2,2':6',2"-terpyridine-platinum (II) complexes in which a N-, halo- or O-nucleophile is the fourth ligand to platinum. Such compounds are potent intercalators of DNA. Unexpectedly, those compounds with a fourth N-ligand and which carry a double positive charge also platinate selectively guanosine residues at N-7 in double stranded DNA. They react with all four free nucleosides found in DNA, but at very different rates. There is no precedent for nucleobases displacing a N-ligand from Pt(II). A new and highly efficient method has been developed for the synthesis of unsubstituted and substituted 2,2':6',2"-terpyridine-platinum(II) complexes.

The most effective compounds have antitumour activity comparable to or better than cisplatin and show little or no cross resistance. Such compounds are more effective than cisplatin against cisplatin-resistant cell lines. Other compounds of this class are most effective against doxorubicin resistant cell lines

Furthermore, this novel class of 2,2':6',2"-terpyridine-platinum (II) complexes possess anti-protozoal activity, against *Leishmania donovani, Trypanosoma cruzi, Trypanosoma brucei, and Plasmodium falciparum.*

2,2':6',2"-Terpyridine-platinum(II) complexes were first reported to bind to double stranded DNA by intercalation over twenty years ago, the duplex unwinding angle being comparable to that found with ethidium bromide [1]. An investigation of the binding of hydroxyethanethiol-2,2':6',2"-terpyridine-platinum(II) (1, R=$SCH_2CH_2OH$) with calf-thymus (ct)-DNA by fiber X-ray diffraction techniques showed the platinum ions in the complex to be distributed along the helix axis in accord with the nearest neighbour exclusion model [2]. The fourth ligand to Pt in all the complexes reported were either thiolate or chloride ions so that the complex bore a single positive charge. The binding association constants with ct-DNA were between $0.23 \times 10^5$ and $2 \times 10^5$ $M^{-1}$, that of the hydroxyethanethiolate complex (**1**,R=$SCH_2CH_2OH$) being $0.83 \times 10^5$ $M^{-1}$. The chloroplatinum complex **1** (R=Cl) had essentially the same binding constant but was shown slowly to form a covalent link to DNA [3]. Recently the hydroxy-platinum complex **1** (R=OH) has been shown to intercalate into DNA with a binding constant of approximately $7 \times 10^4$ $M^{-1}$ and to slowly form a covalent bond with the DNA [4].

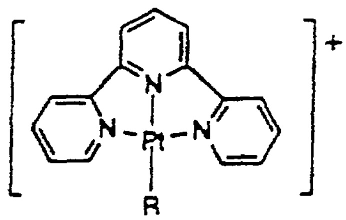

**(1)**

In one aspect the invention provides a 2,2':6',2"-terpyridine Pt(II) complex of the structure

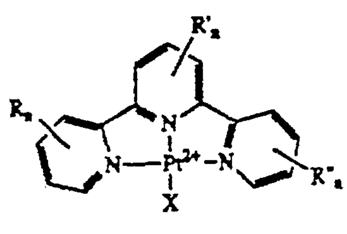

and water-soluble salts thereof,

where X is an aromatic heterocycle or $R^{3}_{,,}$ substituted aromatic heterocycle linked to Pt through N, or a nitrile ($R^4CN$), an amine ($R^5NH_2$), an alcohol ($R^5OH$) ammonia or water linked to Pt through N or O, or a halide ion,
R, R' and R" are the same or different and each is H, alkyl, aryl, aralkyl, alkaryl, acyl, halogen, haloalkyl, haloaryl, hydroxyalkyl, hydroxyaryl, aminoalkyl, aminoaryl, primary, secondary or tertiary amine, hydrazine, alkylhydrazine, alkoxy, aralkoxy, nitrile, ester, amide, nitro, azide or aziridino, or is a covalently linked chain which is joined to at least one other complex of the above structure so as to form a dimeric or oligomeric species,
$R^3$ is a positively charged group or is defined as R, R' and R",

each of $R^4$, $R^5$ and $R^6$ is alkyl, aryl, aralkyl or alkaryl or is a covalently linked chain which is joined to at least one other complex of the above structure so as to form a dimeric or oligomeric species,
and n is 1, 2 or 3,
provided that when each of R, R' and R" is H, then X is not an imidazolium containing ligand.

Substituents may preferably be at the 4'-position of the terpyridine system and/or at the 3 or preferably 4 position of a pyridine ring at X. These substituents may be covalently linked by rigid or flexible chains of indeterminate length by which two or more of the structures may be joined to form dimers or oligomers. Dimeric and oligomeric species may preferably be formed through one or more of $R^3$, $R^4$, $R^5$ and $R^6$, e.g. $R^3$-$R^3$, $R^4$-$R^4$, $R^5$-$R^5$, $R^6$-$R^6$, $R^3$-$R^4$, $R^3$-$R^5$, $R^3$-$R^6$, $R^4$-$R^5$, $R^4$-$R^6$ or $R^5$-$R^6$.
$R^3$ may be a positively charged group such as an N-alkylated pyridine, an N-alkylated aromatic heterocycle or a quaternary ammonium salt.
Reference is directed to the accompanying drawings, in which Figure 1 is a graph of relative total carcinoma volume against time.
Examples of complexes in accordance with the invention are shown below.

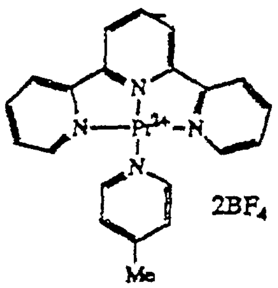

A; Mr=695.09

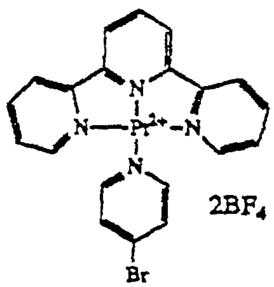

$A_1$; Mr=759.96

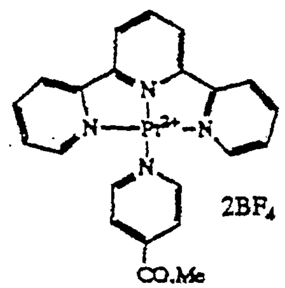

$A_2$; Mr=723.08

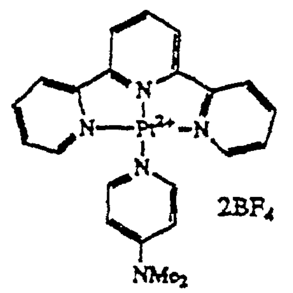

$A_3$; Mr=724.13

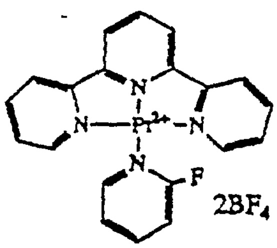

$A_4$; Mr=699.06

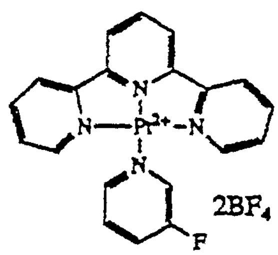

$A_5$; Mr=699.06

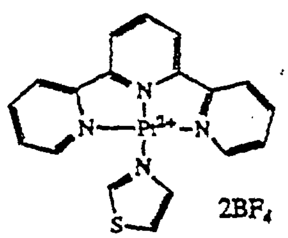

$A_6$=; Mr=687.09

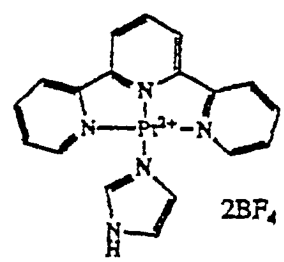

$A_7$; Mr=670.05

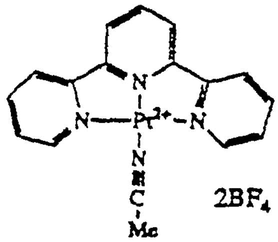

$A_8$; Mr=643.02

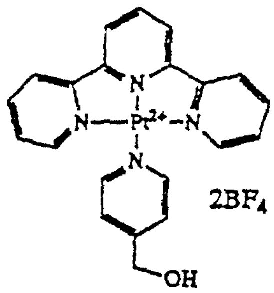

$A_9$; Mr=711,09

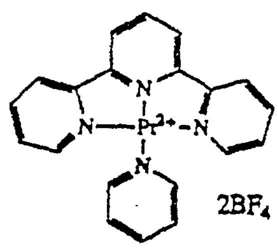

$A_{10}$; Mr=681.06

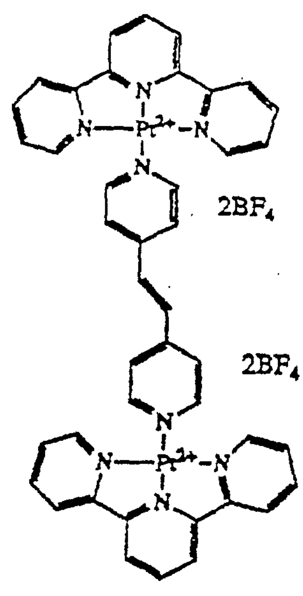

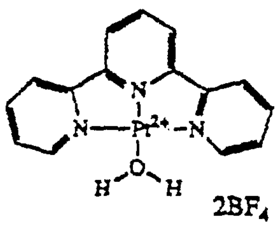

$A_{11}$; Mr=619.98

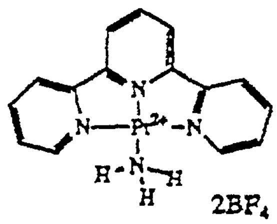

$A_{12}$: Mr=618.99

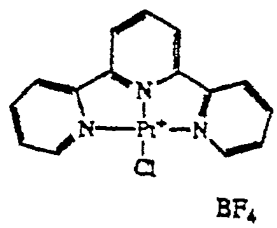

$A_{13}$; Mr=535.3

$A_{14}$; Mr=1314.18

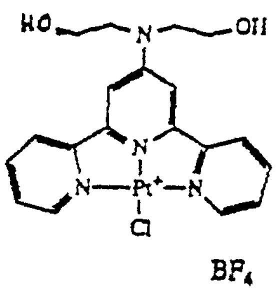

D; Mr=653.74

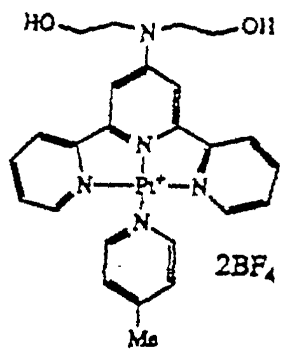

E; Mr=798.22

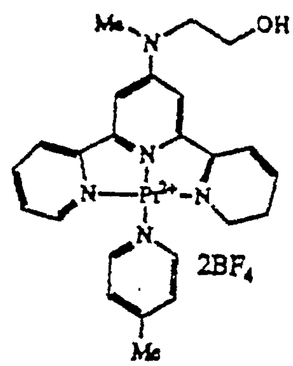

O; Mr=768.19

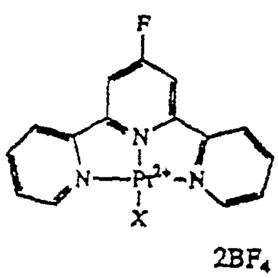

$Y_2$; X= MeCN, Mr=661.01

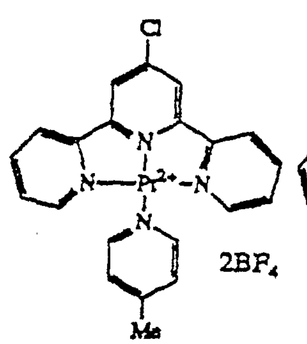

I; Mr=729.54

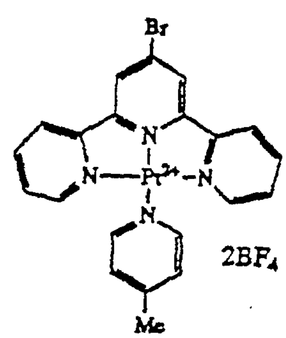

M; Mr=774.17

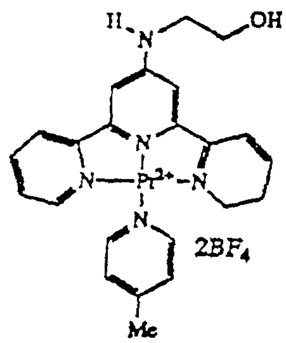

U; Mr=754.17

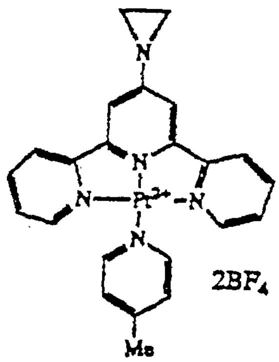

T; Mr=736.16

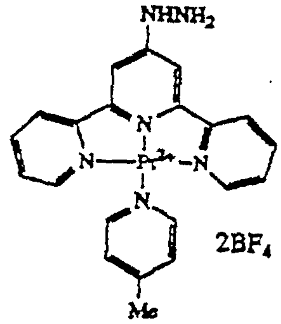

R; Mr=725.13

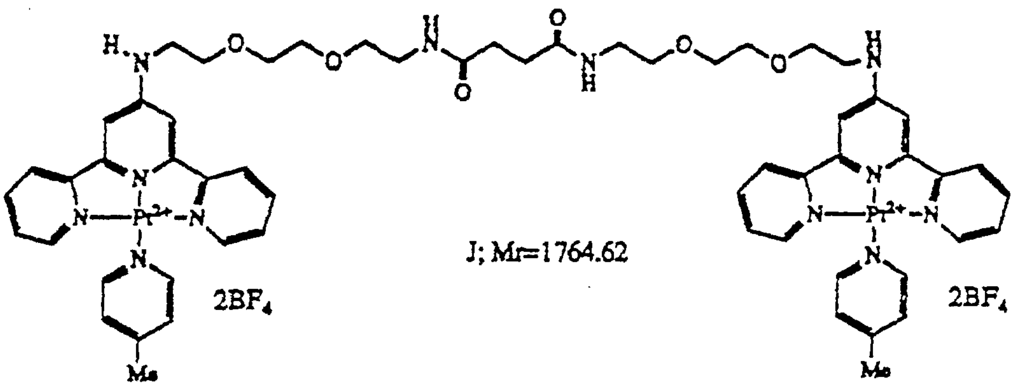

J; Mr=1764.62

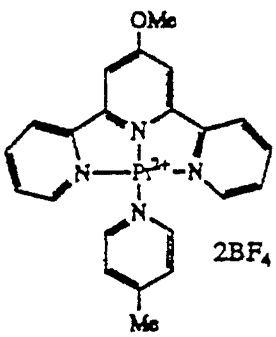

N; Mr=725.12

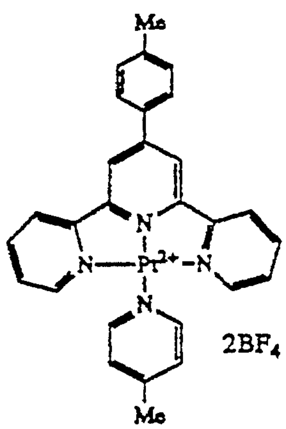

P; Mr=785.22

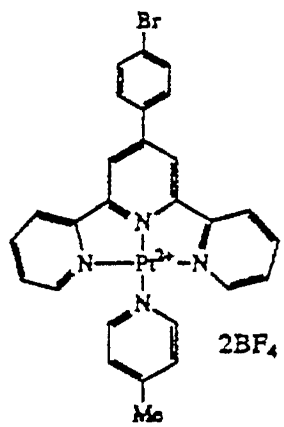

Q; Mr=850.09

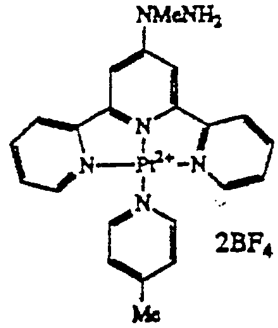

S; Mr=739.16

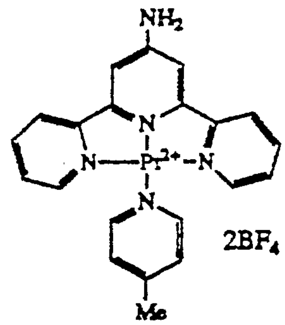

X; Mr=710.11

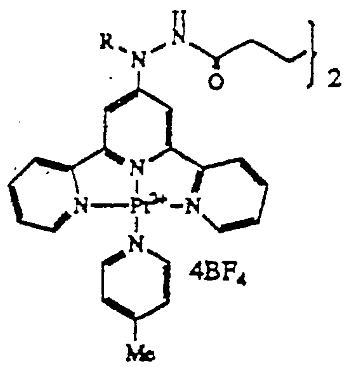

V, R=H; Mr=1560.38
W,R=Me; Mr=1588.43

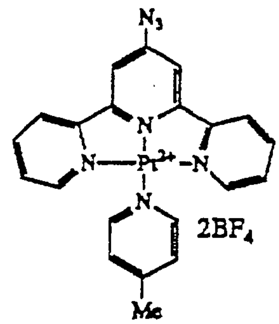

Z, Mr=736.1

**DNA Binding Properties**

In the expectation that a 2,2':6',2"-terpyridine-platinum(II) complex which retains a double positive charge would bind more effectively to DNA, 4-picoline-2,2':6',2"-terpyridine-platinum(II) (**A**), was prepared and its interaction with DNA investigated.

4-Picoline-2,2':6',2"-terpyridine-platinum(II) (**A**) was shown in a ligation assay to unwind and so intercalate into DNA. Circular dichroism was used to determine an equilibrium binding constant of approximately $2x10^7$ $M^{-1}$ for the most stable binding mode of 4-picoline-2,2':6',2"-terpyridine-platinum(II) to poly[d(A-T)$_2$] with a site size of about 4 base pairs, and about $1x10^6$ $M^{-1}$ for a second binding mode with a site size of about 2 base pairs. Fluorescence spectroscopy provided further evidence for the strong equilibrium binding constant of 4-picoline-2,2':6',2"-telpyridine-platinum(II) in that it displaces ethidium bromide bound to DNA. The double positive charge on 4-picoline-2,2':6',2"-terpyridine-platinum(II), together with the intercalative binding mode is probably responsible for the large binding constant.

Attempts to obtain crystals suitable for X-ray analysis of the complex of 4-picoline 2,2':6',2"-terpyridine-platinum(II) (**A**) with the self-complementary oligonucleotide d(CpGpTpApCpG) were unsuccessful. This oligonucleotide was chosen because of the availability of the high resolution structure of its complex with daunomycin, an intercalator of DNA with antitumour activity [5]. Good crystals were obtained, however, from a solution of 4,4'-vinylenedipyridine bis[2,2':6',2"-terpyridine platinum (II)] (**A**$_{14}$) and the oligonucleotide d(CpGpTpApCpG) but the crystals did not diffract X-rays to high resolution. From a series of NMR experiments it became clear that some nucleosides, especially guanosine, were able to slowly displace 4-picoline from 4-picoline 2,2':6',2"-terpyridine-platinum(II) (**A**) and the 4,4'-vinylenedipyridine linker from 4,4'-vinylenedipyridine bis[2.2':6'.2"-terpyridine platinum (II)] (**A**$_{14}$). Although it is well established that purine bases, especially guanine, displace chloride ion from Pt(II) derivatives, the best known example being *cis*platin {*cis*[$Pt(NH_3)_2Cl_2$]) [6], there does not appear to be any previous report of the displacement of a N-ligand at Pt(II) by a nucleobase. indeed it has recently been reported that *cis*-[$Pt(NH_3)_2$(pyridine)Cl]$^+$ forms mono functional adducts with DNA by displacing $Cl^-$, the pyridine ligand being perfectly stable [7]. A mass spectrometric investigation,

including collisional-induced dissociation tandem mass spectrometry, revealed that the self-complementary oligonucleotide d(CpGpTpApCpG) had been platinated at N-7 of the 3'-terminal guanosine residue. In the light of this entirely unexpected observation the reaction of nucleosides with 4-picoline 2,2':6',2"-terpyridine-platinum(II) (**A**) was investigated.

All the nucleosides studied (A, G, dA, dG, dC and dT) were found to react with (**A**) to give platinated nucleosides. Guanosine and 2'-deoxyguanosine reacted with (**A**) in the most straightforward manner and are platinated at N-7. The adenine nucleosides are bis-platinated at N-1 and N-6 and 2'-deoxycytosine is bis-platinated at N-3 and N-4. The product from deoxythymidine has not been characterised. The bis-platinated nucleosides are totally unexpected, no intermediate monoplatinated species being detected. Although the sites platinated on dA and dC are not available in double stranded DNA because of Watson Crick base-pairing, these compounds may have biologically useful properties if they are incorporated into DNA during replication. What they do show is that N-7 of adenine, which is the only nucleophilic site of this nucleobase exposed in double stranded DNA, is considerably less reactive that the N- 1/ N-6 sites which in turn are less reactive than the N-7 site in dG, so providing an explanation of the specificity of platination of dG by (**A**) in the self-complementary oligonucleotide d(CpGpTpApCpG).

**Antitumour Activity**

In the light of the above observations a number of unsubstituted and substituted 2,2':6',2"-terpyridine Pt(II) complexes were prepared and their antitumour activity investigated. The compounds were evaluated for *in vitro* cytotoxicity against five human ovarian carcinoma cell lines which included two selected for resistance to cisplatin (CHIcis$^R$ and A2780cis$^R$) and one for resistance to doxorubicin (CHIdox$^R$). The compounds were exposed to cells for 96 h and growth inhibition assessed using the sulforhodamine B protein staining assay. The $IC_{50}$ values (in μM) are shown in Table 1 and Table 2. Cisplatin was included for comparison.

The effect of the fourth ligand to platinum is seen from the data in Table 1.

Compound (**A**) showed surprisingly variable antitumour activity (the mean value being presented). The potency of this compound may be particularly influenced by the status of the cells and the storage and dilution conditions used. The related dimer (**A$_{14}$**) is very effective against CHI and A2780 cell lines and is significantly better than cisplatin against the corresponding cisplatin-resistant cells (CHIcis$^R$ and A2780cis$^R$) indicating a lack of or low level of cross resistance. The effectiveness of compound (**A$_{14}$**) may be due to the electrostatic repulsion between the two Pt(II) leading to facile nucleophilic displacement at Pt(II).

The effects of substituents at the 4'-position of the 2,2':6',2"-terpyridine tridentate ligand are shown in Table 2. It would appear that large and electron donating substituents as in compounds (**D**) and (**E**) lead to a significant loss of activity in general although with SKOV3 compound (**E**) is remarkably effective. All bisintercalators with flexible linkers generated through the 4'-position e.g. compounds (**J**), (**V**) and (**W**) have proved to be ineffective. However, introducing the 4'-chloro group into the 2,2':6',2"-terpyridine tridendate ligand as in compound (**I**) provides an effective increase in activity against all cell lines. notably the CHI doxorubicin-resistant cell line where an $IC_{50}$ of 0.425 μM was observed.

Compound (**Q**) is effective against CHI and CHI cis$^R$ and especially effective against CHIdox$^R$ cell lines, whereas compound (**T**) is effective against CHI and CHIcis$^R$ cell lines.

In view of the *in vitro* results, compound (**A**) was investigated *in vivo* using the CHI human ovarian carcinoma subcutaneously implanted xenograph. The compound was administered intraperitoneally at weekly intervals for 4 weeks. Two dose levels were used; 30 and 60mg/kg. Tumours were approximately 6-8 mm maximum diameter at the beginning of treatment. Figure 1 shows the mean Relative Tumour Volume (RTV)(e.g. mean from control, 10 animals and treated groups, 5 animals relative to their respective volume at the start of treatment). Two endpoints were determined, the 28 day TIC (the ratio of Treated versus Control volumes at day 28 post initial treatment) and the growth delay (GD: the difference in time taken for Treated *versus* Control groups to double in volume).

Figure 1 shows the effect of compound (**A**), on the Relative Total Volume (RTV) of a CH1 human ovarian carcinoma subcutaneously implanted xenograph, administered at 30 mg/kg and 60 mg/kg intraperitoneally at weekly intervals for 4 weeks. At the lower dose there was a growth delay (GD) 83.1. Compound (**A**) induces a growth inhibitory effect against this tumour, especially at the lower of the two selected doses. There were no deaths due to drug toxicity at either dose. There was, however, evidence, of local peritoneal organ damage at the higher dose which may indicate that at this dose level most of the drug remained in the peritoneal cavity. This would explain why the lower dose was more effective. It seems likely that by using a still lower and more frequent drug schedule (e.g. daily) greater control over tumour growth could be achieved. This could be especially the case with the cisplatin resistant CHI cell line. In view of the known side effects of cisplatin and carboplatin e.g. emesis [8], there is need for improvement in platinum-type antitumour agents. Cisplatin cross-links two nucleobases in DNA (most frequently guanosine)[6], whereas the 2,2':6',2"-terpyridine Pt(II) complexes intercalate into DNA and covalently platinate it, clearly providing a different mechanism of action which is reflected in the observation that it is effective against cisplatin-resistant cell lines.

Compound (**A**) was also administered as a single intraperitoneal injection in water to mice bearing subcutaneously implanted tumours e.g. ADJ/PC6 (according to the standard protocol for evaluating platinum complexes). The compound was toxic at a dose of 100mg/kg but there was no toxic affect at 50mg/kg giving a $LD_{50}$ value of approximately 70mg/kg which is significantly better than cisplatin ($LD_{50}$ approximately 10mg/kg).

In view of the effectiveness of compound (**$A_{14}$**, Table 1) and of compound (I and **Q**, Table 2), a further generation of compounds has been prepared in which the most effective fourth ligand to Pt(II) and the most effective 4'-substituted in the 2,2':6',2"-terpyridine were incorporated into single molecules. Thus compounds (**$I_{14}$**) and (**$Q_{14}$**) have been made and tested. The data on these compounds is shown in Table 3.

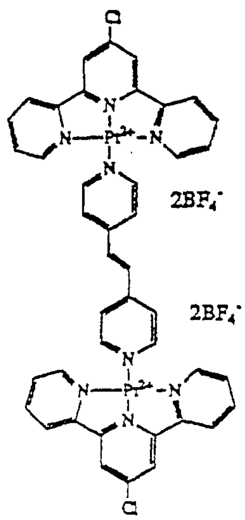

($I_{14}$)

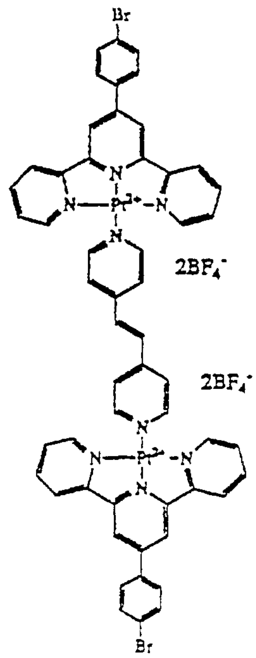

($Q_{14}$)

Compound (**$I_{14}$**) is less effective than compound (**I**) itself, but compound (**$Q_{14}$**) is significantly better than compound (**Q**) against most cell lines and its antitumour activity places it amongst the best compounds of this class."

**Antiparasitic Activity**

The *in vitro* antiprotozoal activity of the compounds were as follows.

<u>SCREEN 1 -PROTOCOL 1</u>

*Leishmania: L. donovani* (MHOM/ET/67/L82) amastigotes, derived from hamster spleen were used to infect mouse peritoneal macrophages in Labtek chamber slides. Infected macrophages were maintained in the presence of the drug at 30 µM and 3 µM in quadruplicate cultures for 5 days at 37°C. Drug activity is measured from % macrophages cleared of amastigotes in treated cultures. Sodium stibogluconate was used as the positive control.

*Trypanosoma cruzi*: Trypomastigotes of *T. cruzi* (MHOM/BR/00/Y) derived from rat L6 myoblast cultures were used to infect mouse peritoneal macrophages in medium containing drugs at 30 µM and 3 µM for 72 hours at 37 C. Drug activity was determined from the % of infected macrophages in treated cultures. Nifurtimox was used as the positive control.

*Trypanosoma brucei; T. brucei* (S427) bloodstream trypomastigotes were cultured in HMI-9 medium at 37°C. Established cultures were incubated with drug at 30 µM and 3 µM for 24 hours. Drug activity was determined at the end of this period by photometric assay. Melarsoprol and pentamidine were used as positive controls.

*Plasmodium falciparum; P falciparum* (chloroquin resistant strain K1) was cultured at 37°C in a 5% suspension of infected erythrocytes (group A $Rh^+$) in complete medium as described by E L Elueze, S L Croft and D C Warhurst, J Antimicrobial Chemotherapy, 1996, 37, 511-518. The *in vitro* antimalarial activity of compounds were likewise determined according to their protocol. Mefloquin was used as the positive control."

SCREEN 2 - PROTOCOL II

The assays follow those outlined in Screen 1- Protocol 1 but include a range of doses in a dilution series from 30μM. Dose response curves were analysed by linear regression and $ED_{50}$ values determined. *T. brucei* numbers/ml are determined using a Coulter Counter.

The data in Table 4 show the % inhibition caused by each of the 2,2':6',2"-terpyridine Pt(II) complexes where the fourth ligand X to Pt(II) is changed. For *Leishmania donovani* and *Trypanosoma cruzi* the most effective compounds are (**$A_{11}$**) and (**$A_{12}$**) i.e. where the fourth ligand is water or ammonia. For *Trypanosoma brucei* the most effective compounds are (**A**) and (**$A_1$**), i.e. where the fourth ligand is 4-methyl-pyridine and 4-bromopyridine.

The data in Table 5 show the effect of changing the substituent at the 4'-position on the 2,2':6',2"-terpyridine Pt(II) complex keeping the fourth ligand X as 4-picoline. For *Leishmania donovani* compounds (**P**) and (**Q**) are remarkably effective at 1 μM. For *Trypanosoma cruzi* compound (**I**), (**R**) and (**S**) were about as effective at 1 μm but for synthetic expediency and because compound (**I**) was the most effective compound for *T. brucei* this was selected for further development. In the light of these results, the following third generation of compounds were prepared and their antiprotozoal activity investigated. The data is shown in Table 6.

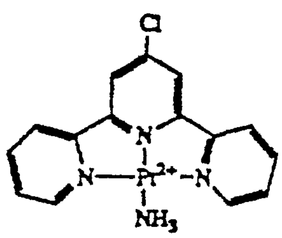

$2BF_4^-$

($I_{12}$)

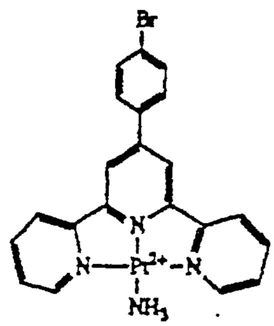

$2BF_4^-$

($Q_{12}$)

Compounds (**P**) and (**Q**) were more effective than sodium stibogluconate ($ED_{50}$ 10 μM, used as the positive control) against *Leishmania donovani*. By using one of the best fourth ligands X (**$A_{12}$** was the most effective compound at 1 μM see Table 4) with two of the best 4' substituents (see Table 5) compounds **$1_{12}$** and **$Q_{12}$** were obtained which completely cleared the cells of *Leishmania donovani* at 1 μM concentration and may well be effective at lower concentrations. Compounds (**$A_{12}$**) and (**I**) as well and (**R**) and (**S**) are significantly more effective against *Trypanosoma cruzi* than the positive control compound, nifurtimox ($ED_{50}$ 3μM). Compound (**$I_{12}$**) may be expected to be even more effective. Compounds (**$Q_{12}$**), (**A**) and (**$A_1$**) all achieve 100% inhibition at 0.1μM against *Trypanosoma brucie.* These compounds are all more effective than melarsoprol and pentamidine which were used as positive controls.

The data in Table 7 shows the effect of a selection of compounds in an assay against a chloroquine resistant strain **K1** of *Plasmodium falciparum.* Compounds (**$A_{11}$**), (**G**), and (**Q**) are all effective in clearing the parasite from infected cells and represent lead compounds for this parasite.

There are a variety of delivery systems which are now being developed which could enhance the effectiveness of the 2,2':6'.2"-terpyrine Pt(II) complexes as antitumour agents, These include the Antibody Directed Enzyme Prodrug Therapy (ADEPT) and Gene Directed Enzyme Prodrug Therapy (GDEPT). Alternatively the effectiveness of antitumour agents can be significantly enhanced by covalently linking them to polymers which are selectively taken up by tumour cells by the EPR (enhanced permeability and retention) effect. If the polymer drug conjugate linkage is susceptible to proteolysis by endosomal or lysosomal proteases then the drug is released intracellularly within the tumour cell leading to an improved therapeutic index.

The 4'-azido-2,2':6',2"-terpyrine Pt(II) complex (**Z**) and bis-intercalators from this Pt(II) complex e.g. (**$Z_{14}$**) where the linker is 4,4'-vinylidenedipyridine, have been developed as tools for molecular biology. A family of bis-intercalators based on the 4'-azido-2,2':6',2"-terpyrine Pt(II) moiety are of general interest since they are able to crosslink two duplexes of DNA which are in close proximity and by photoactivating the azido group covalently cross-link the two DNA duplexes. Since DNA duplexes come close together at sites of replication, genetic recombination and topoisomerase action these molecules will be useful tools to investigate these processes as well as for studying the topology of packaged DNA, e.g. in chromosomes.

**New Synthetic Procedure for the Synthesis of Unsubstituted and Substituted 2,2':6',2" -Terpyridine-platinum (II) Complexes**

The standard procedure for the preparation of 2,2':6',2"-terpyridine-platinum(II) complexes was used initially for the preparation of compound (**A**), but this method is not satisfactory for many of the Pt(II) complexes. A new and highly efficient preparative procedure has been developed.

Most (2,2':6',2"-terpyridine)platinum(II) complexes have been prepared from chloro(2,2':6',2"-terpyridine)platinum(II) chloride (**A$_{13}$**), which can be synthesised by heating $K_2PtCl_4$ and 2,2':6',2"-terpyridine in aqueous solution. The process, however, is slow and typically requires heating for 2-3 days. Furthermore, the reaction rarely proceeds to completion due to the formation of a highly insoluble intermediate $[Pt(terpy)]_2[PtCl_4]$. Using platinum(II) chloride ($PtCl_2$) as the source of platinum and 10 % DMSO in 1:1 water:acetonitrile as the solvent, the reaction time was reduced to a few hours and the product was isolated in nearly quantitative yield.

When the above methods were applied to 4'-substituted 2,2':6',2"-terpyridines, however, only limited success was achieved. For example, the reaction of $PtCl_2$ and 4'-chloro-2,2':6',2"-terpyridine in refluxing acetonitrile-water-DMSO took several days and only a poor yield of the desired product [Pt(Cl-terpy)Cl]Cl was isolated. The method failed completely to give the required product with more hydrophobic terpyridines e.g. 4'-(4-bromophenyl)-2,2':6',2"-terpyridine, even after refluxing for several days.

It was clear that a more rapid and efficient platination method was needed and preferably one which does not require prolonged heating. 1,5-Cyclooctadiene (COD) is a very strong *trans*-labilising ligand and its unfavourable geometry when monocoordinated makes it easily displaced. There are no reports of Pt(COD) complexes being used as starting materials for preparing (2,2':6',2"-terpyridine) platinum (II) complexes.

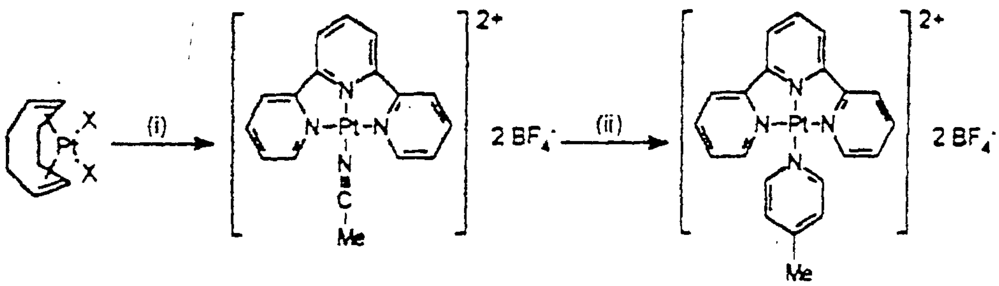

(i) a) 2 eq. $AgBF_4$ / acetone, rt. 2-3 min
b) terpyridine / acetone-acetonitrile, rt. 15-30 min
(ii) 4-picoline / acetonitrile, rt. 2-3 min

*Scheme 1 General method for the synthesis of (2,2':6',2"-terpyridine)platinum (II) complexes*

The method (**Scheme 1**) involves treatment of $Pt(COD)Cl_2$ or $Pt(COD)I_2$ with silver tetrafluoroborate in acetone at room temperature for a few minutes followed by centrifugation to remove the silver halide precipitate. To the clear solution is added a solution of 2,2':6',2"-terpyridine in acetonitrile at room temperature. A pale yellow complex precipitates from the solution after stirring at room temperature for 15-30 min. This acetonitrile complex is washed with acetone to remove any unreacted starting materials then treated with excess of the fourth ligand, e.g. 4-picoline in acetonitrile for a few minutes at room temperature to give 4-picoline 2,2':6':2"-terpyridine platinum (II) tetrafluoroborate (**A**) which precipitates from solution on addition of diethyl ether. Recrystallisation of the complex from acetonitrile by slow diffusion of diethyl ether vapour gave the pure complex in very good yield. Its $^1H$ nmr and mass spectra were identical to the material obtained from the reaction of $[Pt(terpy)Cl]^+$ and 4-picoline in the presence of silver tetrafluoroborate reported in the experimental section. The method has been applied to many other 4'-substituted-2,2':6'2"-terpyridines starting with $Pt(COD)I_2$, to give the desired products which, in all cases, were isolated as the tetrafluoroborate salts in 70-85% yield after recrystallisation. All the complexes gave correct mass and isotope pattern (ES-MS), $^1H$ nmr spectra and satisfactory elemental analyses.

The presence of the COD ligand in the starting material is necessary for labilising the halide ligands and thus initiating the reaction. Similar reactions starting from $Pt(MeCN)_2Cl_2$ and $Pt(DMSO)_2Cl_2$ required heating for several hours and gave the less reactive chloro(2,2':6',2"-terpyridine)platinum(II) complex. The choice of solvent has a significant effect on the success of this method. Methanol may be used in place of acetone but acetonitrile alone gave unsatisfactory results because the silver halides were not precipitated completely even in the presence of excess silver

ion. The presence of acetonitrile enables the intermediate acetonitrile complexes to precipitate from the solution which may be washed to remove any starting materials. Although $Pt(COD)Cl_2$ has been used successfully, $Pt(COD)I_2$ is preferred because the initial halide displacement is faster and the reaction may be monitored visually by the disappearance of the intense yellow colour of $Pt(COD)I_2$. Moreover, $Pt(COD)I_2$ is more soluble in acetone than $Pt(COD)Cl_2$ and AgI has a lower $K_{sp}$ than AgCl.

The method has several important advantages over existing methods. The two-step reaction can be accomplished to give substituted pyridine, pyridine-like heterocyclic amine or ammonia complexes in 1-2 hours and proceeds efficiently at room temperature in non-aqueous solvents which is very important for sensitive substrates. The products can be isolated and purified easily in good yields.

EXPERIMENTAL

By way of example the synthesis of compound (**A**) is described and its reaction with nucleosides. The synthesis of various 4'-substituted 2,2':6',2''-terpyridines and their platinum (II) complexes is also provided.

**Synthesis of 4-picoline-2,2':6':2''-terpyridine-platinum(II) tetrafluoroborate (A).**

A solution of silver tetrafluoroborate (0.20 g, 1.03 mmol, excess) in methanol (5.0 $cm^3$) was added to a solution of $[Pt(terpy)Cl]Cl\text{-}2H_2O$ (0.100g, 0.187 mmol, Aldrich) **1** (R=Cl) in methanol (20.0 $cm^3$). After stirring for 10 min. 4-picoline (0.0255 $cm^3$, 0.262 mmol) was added and the resulting mixture heated to reflux, with light excluded, for 10 h. in order to coagulate the silver chloride which was filtered off while the solution was still hot. On cooling the filtrate yellow crystals formed which are filtered off and washed well with cold methanol. Recrystallisation from methanol afforded 4-picoline-2,2 ':6':2 ''-terpyridine-piatinum(II) tetrafluoroborate (35.1 mg) as bright yellow needles (m.p. > 220°C); $\upsilon_{max}$(KBr disc) 3387br s, 3083br m, 3032br m, 1606s, 1477m, 1453s, 1400m, 1318m, 1056br vs, 776vs and 722m $cm^{-1}$; $\lambda_{max}$ ($\varepsilon$) ($H_2O$) 240 (28800), 269 (19000), 324 (8700) and 339 (15000) nm; $^{\delta}H$ (5OOMHz, $D_2O$) 2.59 (3H, s, -C$\underline{H}_3$) 7.65 (2H, m, 2 x C(5)$\underline{H}$), 7.70 (2H, d, $\underline{J}$ = 6.3 Hz, 2 x C(8)$\underline{H}$), 7.77 (2H, d, $\underline{J}$ = 5.6 Hz, 2 x C(6)$\underline{H}$), 8.34-8.37 (4H, m, 2 x C(4)$\underline{H}$ and 2 x C(3)$\underline{H}$, 8.36 (2H, d, $\underline{J}$ = 9.0 Hz, 2 x C(3')$\underline{H}$), 8.47 (1H, t, $\underline{J}$ = 8.3 Hz, C(4')$\underline{H}$), 8.79 (2H, d, $\underline{J}$ = 6.6 Hz, 2 x C(7)$\underline{H}$); m/z (ESI) 260.5 ([4-picoline-terpyridine-Pt(II)]$^+$, 100%).

4-Picoline-2,2':6':2''-terpyridine-platinum(II) tetrafluoroborate (A) was also prepared in 77% yield (isolated crystalline product) by the new method of synthesis starting with $Pt(COD)I_2$. It was identical with the product described above.

To a suspension of diiodo(1,5-cyclooctadiene)platinum (II) (55mg, 0.10 mmol) in acetone[1,2] (1 ml) in an Eppendorf tube was added silver tetrafluoroborate (40 mg, 0.21 mmol). The resulting mixture was stirred at room temperature until a clear colourless solution with a pale yellow precipitate of AgI obtained (typically 2-3 min) then the precipitate was centrifuged off and discarded. The solution was added to a suspension of 2,2':6',2''-terpyridine (18.7 mg, 0.08 mmol) in acetonitrile (0.5 ml) and the reaction mixture stirred at room temperature for another 30 min. The yellow crystals of the acetonitrile complex formed were collected by centrifugation and washed with acetonitrile and the crystals resuspended in acetonitrile. 4-Picoline (10 µl, excess) was then added and the reaction mixture stirred at room temperature for a few minutes to give a clear solution. The picoline complex was precipitated by addition of diethyl ether and washed with ether. Recrystallisation from acetonitrile or methanol gave compound (**A**) as yellow needles (43 mg, 77%) m.p. >200°C (dec) (Found C, 36.5, H, 2.0, N, 8.2 % $PtC_{21}H_{18}N_4B_2F_8$ requires C, 36.3. H, 2.6, N, 8.1%). $\delta_H$ (200 MHz, $CD_3CN$) 2.64 (3H, s, picoline-Me), 7.66-7.88 (m, 6H, $H_{5.5^*}$, $H_{6.6^*}$ and picoline-$H_{3.5}$), 8.30-8.47 (m, 6H, $H_{5.5^*}$, $H_{6.6^*}$ and picoline-$H_{3.5}$), 8.30-8.47 (m, 6H,, $H_{4.4^*}$, $H_{3.3^*}$ and $H_{3',5')}$, 8.54 (1H, dd, $H_{4'}$), 8.81 (2H, d with broad $^1H\text{-}^{195}Pt$ satellites, picoline-$H_{2,6}$); m/z (ES MS) 260.5 ($M^{2+}$, 100%).

**Notes:**

1. Most other complexes were also prepared by the new method. Indeed most could not have been made by conventional methods.
2. Methanol may be used in place of acetone and gives cleaner products in some cases.
3. Timing varies, depending on the solubility of the terpyridine in the reaction mixture. For highly insoluble terpyridines, longer reaction times are needed to ensure complete reactions.

**Isolation and characterisation of N7-1(2,2':6',2"terpyridine)platinum(II) guanosine complex**

(a) from compound (**A**): Compound (**A**) (13.9 mg, 20 μmol) and guanosine (28.5 mg, 100 μmol) were dissolved in deionised water (1.8 ml). A sodium phosphate buffer pH 5.5 (0.5 M; 200 μL) was added and the solution was incubated at 37 °C for 10 days. The complex was purified on Sephadex G-10. $^1$H NMR of the product after gel filtration on a second column indicated that it is a platinated guanosine but contaminated with some starting materials.

(b) from compound ($\mathbf{A_{13}}$): A solution of [Pt(terpy)Cl]Cl.2$H_2O$ (26.7 mg, 50 μmol) and silver nitrate (17 mg, 100 μmol) in deionised water (1.0 ml) was heated at 70-80°C in a water bath for 2 h. The AgCl precipitate was centrifuged off, the solution added to guanosine (15 mg, 52 μmol), and the solution heated for another 2 h at 70-80°C. After centrifugation. the yellow solution was freeze dried to give the nearly pure complex (contaminated by trace of guanosine) in quantitative yield. $\delta_H$ (500 MHz, 2$H_2O$) 3.82 (1H, dd, *J*= 12.8, 4.1 Hz, CHaHbOH), 3.91 (1H, dd, *J*= 12.7, 2.9 Hz, CHaHbOH), 4.28 (1H, m, H4'), 4.43 (1H, t, *J*=4.8 Hz, H3'), 4.82 (1H, t, *J*=5.0 Hz, H2'), 6.10 (1H, d *J*=4.8 Hz, H1'), 7.65 (2H, m, terpy H5,5"), 7.92 ( 2H, dd *J*=11.5, 5.6 Hz,terpy H6,6"), 8.32-8.36 (6H, m, terpy H3,3", H4,4" and H3',5'), 8.49 (1H, t *J*=5.0 Hz, terpy H4'), 8.95 (1H, s, guanine-H8).; m/z (ES MS) 355.78 (M2+, 100%).

**Isolation and characterisation of N1-[(2,2':6' 2"-terpyridine)platinum(II)]-N6-[(2,2':6',2"-terpyridine)platinum(II)] adenosine and N1-[(2,2':6',2"-terpyridine)platinum(II)]-N6'-[(2,2':6',2"-terpyridine)platinum(II)]I-2'-deoxyadenosine**

(a) from compound (**A**): compound(**A**) (13.9 mg, 20 μmol) and adenosine (26.8 mg, 100 μmol) were dissolved in 1.8 ml of deionised water (1.8 ml). Sodium phosphate buffer pH 5.5 (0.5 M, 200 μL) was added and the solution was incubated at 37°C for 10 days. The deep orange solution was first purified on a column of Sephadex G-15 followed by chromatography on a column of Sephadex G-10. The orange fractions were collected and freeze dried to give the product as a red powder (~5 mg) $\delta_H$ (500 MHz, 2$H_2O$) 3.96 (2H, m, $CH_2OH$)), 4.39 (1H, m, H4'), 4.50 (1H, dd, *J*=5.6, 3.5 Hz, H3'), 4.90 (1H, dd, *J*=6.0, 5.5 Hz, H2'), 6.23 (1H, d *J*=6.2. Hz, H1'); 7.50 (4H, 2xt, terpyl-H5,5" and terpy2-H5,5"), 8.05 (2H, dd, *J*=10.1, 4.6 Hz, terpy 1-H6,6"), 8.10 (4H, 2xd, terpy 1-H3,3" and terpy2-H3,3"), 8.15 (4H, 2xd, terpyl-H3',5' and terpy2-H3',5'), 8.25 (4H, 2xt, terpy1-H4,4" and terpy2-H4,4"), 8.42 (2H, br m, terpy2-H6,6"), 8.48 (2H, 2xt, terpy1-H4' and terpy2-H4'), 8.55 (1H, s, adenine-H8), 8.85 (1H, s, adenine-H2).

(b) from compound ($\mathbf{A_{13}}$): A solution of [Pt(terpy)Cl]Cl.2$H_2O$ (53.5 mg 100 μmol) and silver nitrate (35.0 mg 200 μmol) was heated at 70-80°C in a water bath for 30 min and the AgCl precipitate was centrifuged off. To the solution was added adenosine (28 mg, 100 μmol) and potassium carbonate (14 mg, 100 mmol), and the solution heated for another 2 h at 70-80°C. After centrifugation, the deep red solution was neutralised by addition of $NaH_2PO_4$ and the complex precipitated with ammonium hexafluorophosphate. The orange precipitate was filtered off, washed with water and air dried. Re-precipitation of the hexafluorophosphate salt from acetonitrile-ether gave the complex as an orange-brown powder (64.6 mg, 84 %), δH (200 MHz, $C^2H_3CN$) 3.65-3.95 (4H, m, C$\underline{H_2}$OH and 2 x OH), 4.20 (1 H, m, H4'), 4.35 (1 H, br m, OH), 4.65 (1H, dd, *J*=10.5, 4.0 Hz, H3'), 4.80 (1H, m, H2'), 6.00 (1H, d, *J*=6.6 Hz, H1'), 7.08 (1H, br s, adenine N6$\underline{H}$, 7.40 (4H, m, 2 x terpy-H5,5"), 7.80-8.05 (1OH, m, terpy1-H6,6", 2 x terpy-H3,3", 2 x terpy-H3',5'), 8.15 (4H, m, 2 x terpy-H4,4"), 8.31 (1H, s, adenine-H8), 8.30-8.42 (4H, m, terpy2-H6,6" and 2 x terpy-H4'), 8.60 (1H, s, adenine-H2); m/z (ES MS) 374.3 (M3+, 100%), 347.8 {[M-Pt(terpy)+H]2+}, 330.2 {[M-ribose]3+}. Similarly the 2'-deoxyadenosine complex was synthesised on the same scale, following counterion exchange and re-precipitation (69.5 mg, 90 %), δH (500 MHz, $C^2H_3CN$) 2.50 and 2.88 (2H, 2 x m, 2 x H2'), 3.50 (1H, br m, OH), 3.75 (2H, m, C$\underline{H_2}$OH), 4.10 (1H, m, H4'), 4.43 (1H, m, H3'), 4.62 (1H, brm, OH), 6.50 (1H,dd, *J*=8.3,5.9Hz,Hl'), 7.02(1H,br,s, adenineN6$\underline{H}$), 7.40(4H,m, 2 x terpy-H5,5"), 7.80-8.05 (1OH, m, terpy1-H6,6", 2 x terpy-H3,3", 2 x terpy-H3',5'), 8.15.(4H, m, 2 x terpy-H4,4"), 8.30 (1H, s, adenine-H8), 8.30-8.40 (4H, m, terpy2-H6,6" and 2 x terpy-H4'), 8.60 (1H, s, adenine-H2); m/z (ES MS) 369.0 (M3+, 100%), 339.8 {[M-Pt(terpy) +H]2+}, 330.2 {[M-deoxyribose)3+}

**Isolation and characterisation of N3-[(2,2':6',2"-terpyridine)platinum(II)]-N4-[(2 ,2 ':6',2"-terpyridine)platinum (II)]-2'-deoxycytidine**

(a) from compound (**A**): as for the guanosine and adenosine complex starting from [Pt(terpy)(4-picoline)](BF4)2 (**A**) (13.9 mg, 20 µmol) and 2'-deoxycytidine (22.6 mg, 100 µmol). The product which precipitated from the solution upon addition of a saturated aqueous solution of sodium tetrafluoroborate, was collected by centrifugation and recrystallised from water (5 mg, 37 %), mp>200°C; (Found: C, 34.0; H, 2.1; N, 8.8. $C_{39}H_{34}N_9O_4Pt_2B_3F_{12}$.0.3$NaBF_4$ requires C, 34.0, H, 2.5, N, 9.1 %); δH (200 MHz, $^2H_2O$) 2.52 (2H, m, H2'), 3.88 (2H, m, C$\underline{H}_2$OH), 4.12 (1H, m, H4'), 4.55 (1H, m, H3'), 6.42 (1H, t, *J*=6.7 Hz, H1'), 6.60 (1H, d *J*=7.8 Hz, cytosine-H5) 7.50-7.56 (4H, m, 2 x terpy-H5,5"), 7.82 (1H, d, *J*=7.8 Hz, cytosine-H6), 8.0-8.5 (18H, m, terpy-H); m/z (ES MS) 360.95 (M3+, 100%).

(b) from compound (**A$_{13}$**): A solution of [Pt(terpy)Cl]Cl 2$H_2O$ (26.7 mg, 50 µmol) and silver nitrate (17 mg, 100 µmol) in deionised water (1.0 ml) was heated at 70-80°C in a water bath for 2 h. The AgCl precipitate was centrifuged off, the solution added to 2'-deoxycytidine (11.8 mg, 52 µmol), and the solution heated for another 2 h at 70-80 °C. After centrifugation, the product was precipitated by addition of sodium tetrafluoroborate and recrystallised from water to give the complex as red microneedles (25 mg, 70 %). Counterion exchange with ammonium hexafluorophosphate in water provided the red hexafluorophosphate in quantitative yield. δH (500 MHz, $C^2H_3CN$) 2.34 (m, 2H, 2 x H2'), 3.22 and 3.35 (2H, 2 x br s, 2 x OH), 3.78 (1H, dd, *J*= 12.0, 3.6 Hz, C$\underline{H}$aHbOH), 3.84 (1H, dd, *J*= 12.0, 3.3 Hz, CHa$\underline{H}$bOH), 3.96 (1H, dd, *J*= 11.2, 3.6 Hz, H4'), 4.43 (1H, m, H3'), 6.26 (1H, t *J*=ó.3 Hz, H1'), 6.37 (1H, d *J*=7.8 Hz, cytosine-H5), 6.84 (1H, br s, cytosine-N6$\underline{H}$), 7.44-7.54 (4H, m, 2 x terpy-H5.5"), 7.88-8.00 (9H, m, 2 x terpy-H3,3", 2 x terpy-H3',5' and cytosine-H6). 8.13-8.21 (6H, m. 2 x terpy-H4,4" and terpyl-H6.6"). 8.27 (2H, ddd. J= 7.3, 5.5 and 1.3 Hz, terpy2-H6,6"), 8.33 and 8.38 (2H, 2 x t, *J*=8.1 and 8.2 Hz, 2 x terpy-H4'). 4'-Chloro-2.2':6'.2"-terpyridine [9] and 4'-bromo-2.2':6'.2"-terpyridine [10] were prepared by literature methods.

**4'-(N,N-bis(2-hydroxyethyl)amino)-2,2':6',2"-terpyridine.**

$FeCl_2 \cdot 4H_2O$ (1.6 g, 8.1 mmol) and diethanolamine (23.563 g, 224.11 mmol, 60 equiv.) were added to a solution of 4'-chloro-2.2':6'.2"-terpyridine (1 g, 3.73 mmol) in ethanol / methanol 1 /1 (80 ml). The purple solution was refluxed for 60 h and then filtered through celite. The solvent was evaporated *in vacuo* and the resulting viscous residue adjusted to pH 3 by dropwise addition of a solution of 2 M hydrochloric acid. The acidic solution was treated with $[NH_4][PF_6]$ (2g) and $[Fe((HOCH_2CH_2)_2Nterpy)_2][PF_6]_2$ precipitated as a purple solid which was collected by centrifugation, washed with 0.1 M $[NH_4][PF_6]$ and ether, and then air-dried. The solid was then dissolved in a solution of acetonitrile / 1M NaOH (20 ml) and the solution so obtained stirred under $O_2$ until the purple colour had disappeared (ca. 20 h). The brown reaction mixture was filtered through celite and the solid residue washed with acetonitrile. The combined filtrate and extracts were then concentrated *in vacuo* and the white solid so obtained was collected by filtration and recrystallised from dichloromethane to afford white needles of 4'-(N,N-bis(2-hydroxyethyl)amino)-2,2':6',2"-terpyridine (352 mg, 31%). mp 171-172°C. $\delta_{1H}$ (500 MHz, $CD_3OD$) : 8.61 (*dd*, $^4J$(6,4) = 1.4, $^3J$(6,5) = 4.5, 2H, H-C(6), H-C(6")); 8.52 (d, $^3J$ (3,4)=8.0, 2H, H-C(3), H-C(3")); 7.93 (*dt*, $^4J$(4,6) = 1.8, $^3J$(4,3) = $^3J$(4,5) = 7.7, 2H, H-C(4), H-C(4")); 7.68 (s, 2H, H-C (3'), H-C(5')); 7.42 (*ddd*, $^4J$(5,3) = 1.2, $^3J$(5.6) = 4.9, $^3J$(5,4) = 7.5, 2H, H-C(5) H-C(5")); 3.85 (*t*, $^3J_{vic}$ = 5.9, 4H, $\underline{H}_2$-COH); 3.75 (*t*, $^3J_{vic}$ = 5.9, 4H, $\underline{H}_2$-CNterpy). $\delta_{13C}$ (50.3 MHz, $CD_3OD$) : 156.92 (1C, C(4')); 155.66 (2C, C(2) or C(2'), C(6') or C(2")); 155.25 (2C. C(2) or C(2'), C(6') or C(2")); 148.55 (2C, C(, C(6), C(6")); 137.49 (2C, C(4), C(4")); 123,49 (1C, C(5), C(5")); 121.94 (1C, C(3), C(3")); 103.91 (1C, C(3'), C(5')); 58.72 (1C, $H_2\underline{C}OH$; 52.86 (1C, $H_2\underline{C}$Nterpy). m/z (ESI) : 377 ($MH^+$). Anal. Calcd, for $C_{19}H_{20}N_4O_2$ : C, 67.84; H, 6.00; N, 16.65. Found C, 67.59; H, 6.02; N, 16.41.

*Isolation and characterisation of the two alkoxy side products.* The dichloromethane filtrate remaining from the recrystallisation was evaporated *in vacuo* and the solid residue chromatographed on an alumina preparative plate using P.E. / EtOAc 7 / 1 as eluant and carrying out two elutions to allow a better separation. Three bands were obtained and identified as 4'-chloro-2.2':6'.2"-terpyridine, 4'-methoxy-2,2':6',2"-terpyridine and 4'-ethoxy-2,2':6',2"-terpyridine .

**4'-Methoxy-2,2':6',2"-terpyridine.** mp 56-57°C. TLC (alumina, P.E. / EtOAc 3 /1) : Rf = 0.53. $\delta_{1H}$ (200 MHz, $CDCl_3$) : 8.71 (d, $^3J$(6,5) = 4.7, 2H, H-C(6), H-C(6")); 8.64 (*d*, $^3J$(3,4)=8.0, 2H, H-C(3), H-C(3")); 8.04 (*s*, 2H, H-C(3'), H-C(5'));7.87 (*dt*, $^4J$(4,6) = 1.8, $^3J$(4,3) = $^3J$(4,5) = 7.8, 2H, H-C(4), H-C(4")); 7.34 (*ddd,* $^4J$(5,3) = 1.1, $^3J$(5,6) = 4.8, $^3J$ (5,4) = 7.5, 2H, H-C(5), H-C(5")); 4.05 (s, 3H, $\underline{H}_3$-COterpy). $\delta_{13C}$ (50.3 MHz, $CDCl_3$) : 168.15 (1C, C(4')); 157.35 (2C, C(2) or C(2'), C(6') or C(2")); 156.33 (2C, C(2) or C(2'), C(6') or C(2")); 149,28 (2C, C(, C(6), C(6")); 137.04 (2C, C(4), C(4")); 124.03 (1C, C(5), C(5")); 121.54 (1C, C(3), C(3")); 107.05 (1C, C(3'), C(5')); 55.55 (1C, $H_3\underline{C}$Oterpy). m/z (ESI) : 264 ($MH^+$). This compound can be prepared in excellent yield by methanolysis of 4'-chloro-2.2':6'.2"-terpyridine activated by $FeCl_2$.4$H_2O$.

**4'-Ethoxy-2,2':6',2"-terpyridine.** mp 85-86°C. TLC (alumina, P.E. / EtOAc 3 / 1) : $R_f$ = 0.59. $\delta_{1H}$ (200 MHz, $CDCl_3$) : 8.70 (*d*, $^3J$(6,5) = 4.1, 2H, H-C(6), H-C(6")); 8.63 (*d*. $^3J$(3,4)=8.1, 2H, H-C(3), H-C(3")); 8.02 (*s*, 2H, H-C(3'),

H-C(5')); 7.86 (*dt*, $^4J(4,6)$ = 1.8, $^3J(4,3)$ = $^3J(4,5)$ = 7.3, 2H, H-C(4), H-C(4")); 7.34 (*ddd,* $^4J(5,3)$ = 1.2, $^3J(5,6)$ = 4.8, $^3J(5,4)$ = 7.5, 2H, H-C(5), H-C(5")); 4.32 (*q*, 2H, $^3J_{vic}$ = 7.1, 2H, $\underline{H_2}$-COterpy); 1.50 (*t*, $^3J_{vic}$ = 6.9,$\underline{H_3}$-$CCH_2$). m/z (ESI) : 278 ($MH^+$). This compound can be prepared in excellent yield by ethanolysis of 4'-chloro-2.2':6'.2"-terpyridine activated by $FeCl_2.4H_2O$.

**4'-(*N*-2-Hydroxyethyl-*N*-methyl)amino-2,2':6',2"-Terpyridine**

A solution of 4'-chloro-2,2':6',2"-terpyridine (1.61g, 6mmol) in 20ml of dichloromethane was added to a solution of $FeCl_2.4H_2O$ (1.35g, 6.8mmol) in 100ml of isopropanol. An excess of methylaminoethanol (20ml) was then added to the purple solution. The mixture was heated to reflux for 22 hr under an atmosphere of argon and with stirring. It was then concentrated by removal of solvent in *vacuo* to leave a viscous purple oil. Addition of $[NH_4][PF_6]$ (1.66g, 10mmol) in 10ml methanol, followed by 50ml diethyl ether, led to the purple gum $[Fe(Me(ClCH_2CH_2)Nterpy)_2][PF_6]$ being precipitated below a clear supernatant liquid. The solid was washed with $Et_2O$ (3x50ml) by decantation, triturated with $H_2O$ (40ml), then collected by filtration and further washed with $Et_2O$ (40ml) and $H_2O$ (40ml). The residue was then dissolved in a solution of sodium hydroxide (4g) in 300ml of 1:1 $H_2O$:acetonitrile and stirred under oxygen, at room temperature, until the purple coloration had disappeared. The brown reaction mixture was filtered through celite and the solid residue washed with $H_2O$ (50ml), MeCN (50ml) and DCM (100ml). After concentration of the filtrate *in vacuo* the organic phase was separated, washed with brine (2x40ml), dried over anhydrous magnesium sulphate, filtered and evaporated to dryness. The resulting powdery solid was recrystallised from acetone/P.E. (40-60°C) to give 4'-(*N*-2-hydroxyethyl-*N*-methyl)amino-2,2':6',2"-terpyridine (1.44g, 78%): m.p. 148-150°C ;$\nu_{max}$ (KBr)/$cm^{-1}$ 3219(m.br), 2880-2820(m,br), 1609(m), 1592(s), 1567(s), 1459(m), 1412(m). 1228(w), 1055(s), 1006(m) 851(w), 790(s); $\delta_{1H}$ (200 MHz; $CDCl_3$) 8.65 (d, $^3J(6,5)$=4.5, 2H, H-C(6), H-C(6")); 8.60 (d, $^3J(3,4)$=8.5, 2H, H-C(3), H-C(3")); 7.83 (dt, $^3J(4,5)$=7,77, $^4J(4,6)$=1.8, 2H, H-C(4), H-C(4")); 7.78 (s, 2H, H-C(3'), H-C(5')); 7.30 (ddd, $^3J(5,4)$=7.4, $^3J(5,6)$=4.8, $^4J(5,3)$=1.1, 2H, H-C(5), H-C(5")); 3.9 (t, $^3J_{vic}$=5.8, 2H, terpyNMe.$CH_2C\underline{H_2}OH$); 3.7 (t, $^3J_{vic}$=5.8, 2H, terpyNMe. $C\underline{H_2}CH_2OH$); 3.2 (s, 3H, terpyN$C\underline{H_3}$.$CH_2CH_2OH$).

**4'-(1-Methylhyrazino)-2,2':6',2"-Terpyridine**

4'-Chloro-2,2':6',2"-terpyridine (148mg, 0.55mmol) was dissolved in isobutanol (3ml) with warming. Excess methylhydrazine (0.8ml) was added. The mixture was then heated to reflux, with stirring, for 28 hrs. On cooling white needles crystallised out of solution. The solid was collected by filtration and washed with a few drops of diethyl ether to yield analytically pure 4'-(1-methylhyrazino)-2,2':6',2"-terpyridine (132mg, 86%): m.p 217-219°C; (Found: C, 69.6; H, 5.05; N, 25.2. $C_{16}K_{15}N_5$ requires C, 69.3; H, 5.4; N, 25.2); $\nu_{max}$(KBr)/$cm^{-1}$ 3323(m), 3100-2700(w,br), 2359(w,br), 1562(s), 1582(s), 1470(s), 1408(s), 1093(m), 1005(m), 826(w); $\delta_{1H}$ (200 MHz; $CDCl_3$) 8.70 (d, $^3J(6,5)$=4.7, 2H, H-C(6), H-C(6")); 8.64 (d, $^3J(3,4)$=7.98, 2H, H-C(3), H-C(3")); 7.98 (s, 2H, H-C(3'), H-C(5')); 7.85 (dt, $^3J(4,5)$=7.56, $^4J(4,6)$=1.77, 2H, H-C(4), H-C(4")); 7.33 (ddd, $^3J(5,4)$=7.4, $^3J(5,6)$=4.8, $^4J(5,3)$=1.2, 2H, H-C(5), H-C(5")); 4.06 (s, 2H, terpyN-Me.$N\underline{H_2}$); 3.40 (s, 3H, terpyN$C\underline{H_3}$.$NH_2$); m/z (ESI)= 278 (100% , $[MH]^+$).

**4'-Hydrazino-2,2':6',2"-Terpyridine**

4'-Chloro-2,2':6',2"-terpyridine (600mg, 2.2mmol) was dissolved in isobutanol (12ml) with warming. Excess hydrazine (4ml) was added. The mixture was then heated to reflux under argon, with stirring, for 30 hours. On cooling white crystals precipitated out of solution. These were collected by filtration and washed with a few drops of water to yield analytically pure 4'-hydrazino-2,2':6',2"-terpyridine (439mg, 74%): m.p 195-197°C; (Found: C, 68.47; H, 4.61; N, 26.58. $C_{15}H_{13}N_5$ requires C, 68.43; H, 4.98; N, 26.60); $\nu_{max}$(KBr)/$cm^{-1}$ 3329(w,br), 3277(s), 3181(m), 1644(w), 1605(m), 1585(s), 1565(s), 1466(m), 1403(m), 1229(w), 1069(w), 996(m), 864(m); $\delta_{1H}$ (200 MHz; $CDCl_3$) 8.69 (d, $^3J(6,5)$=4.6, 2H, H-C(6), H-C(6")); 8.63 (d, $^3J(3,4)$=8.0, 2H, H-C(3), H-C(3")); 7.87 (s, 2H, H-C(3'), H-C(5')); 7.86 (dt, $^3J(4,5)$=$^3J(4,3)$=7.7, $^4J(4,6)$=1.6, 2H, H-C(4), H-C(4")); 7,34 (ddd, $^3J(5,4)$=7.4, $^3J(5,6)$=4.9, $^4J(5,3)$=1.3, 2H, H-C(5), H-C(5")); 5.81 (s, br, 1H, terpyN$\underline{H}$.$NH_2$); 3.83 (s, br, 2H, terpyNH. $N\underline{H_2}$); m/z (ESI) = 264.26 (100%, $[MH]^+$).

**4'-(*N*-2-Hydroxyethyl)amino-2,2':6',2"-Terpyridine**

A solution of 4'-chloro-2,2':6',2"-terpyridine (137mg, 0.5mmol) in dichloromethane (2ml) was added to a solution of $FeCl_2.4H_2O$ (112mg, 0.6mmol) in isopropanol (10ml). An excess of ethanolamine (1ml) was then added to the purple solution. The mixture was heated to reflux for 19 hours under an atmosphere of argon and with stirring. Work up proceeded as above. The resulting powdery solid recrystallised from acetone/P.E. (40-60°C) to give 4'-(*N*-2-hydroxyethyl)amino-2,2':6',2"-terpyridine (90mg, 60%): m.p. 145-147°C; (Found: C, 70.0; H, 5.2; N, 18.9. $C_{17}H_{16}N_4O$ requires C, 69.9; H, 5.5; N, 19.2); $\nu_{max}$(KBr)/$cm^{-1}$ 3424(m), 3200(m,br), 2935(w), 1608(m), 1588(s), 1565(m), 1480(m), 1465(m), 1400(w), 1083(m), 989(m); $\delta_{1H}$.(200 MHz; $CDCl_3$) 8.67 (d, $^3J(6,5)$=4.8, 2H, H-C(6), H-C(6")); 8.61 (d, $^3J(3,4)$=7.89, 2H, H-C(3), H-C(3")); 7.85 (dt, $^3J(4,3)$=$^3J(4,5)$=7.7, $^4J(4,6)$=1.8, 2H, H-C(4), H-C(4")); 7.71 (s, 2H, H-C(3'), H-C(5')); 7.32 (ddd, $^3J(5,4)$=7,6, $^3J(5,6)$=4.8, $^4J(5,3)$=1.3, 2H, H-C(5), H-C(5")); 4.92 (s, 1H. terpyN$\underline{H}$.$CH_2CH_2OH$); 3.92 (t, $^3J_{vic}$=5.0, 2H, terpyNH.$CH_2C\underline{H_2}OH$); 3.56 (q, $^3J_{vic}$=5.4, 2H, terpyNH.$C\underline{H_2}CH_2OM$); m/z (ESI)=293.26 (100%, $[MH]^+$).

**4'-(*N*-2-Chloroethyl)amino-2,2':6',2"-Terpyridine**

A solution of 4'-(*N*-2-hydroxyethyl)amino-2,2':6',2"-terpyridine (880mg, 3mmol) in thionyl chloride (8ml) was kept at room temperature for 20 hours. Excess thionyl chloride was removed *in vacuo* to leave a green glassy solid. This solid was suspended in dichloromethane (40ml) and dissolved, with effervescence, when washed with saturated aq. sodium bicarbonate solution (2x25ml). The aq. phase was separated and reextracted with further DCM. The organic extracts were then combined and dried over anhydrous magnesium sulphate. Filtration and evaporation of the solvent *in vacuo* gave a solid which was purified by elution through a column of activity IV neutral alumina. Initially 100% DCM then 50:1 DCM:methanol were used as eluant. Impurities remained on the base line. Removal of solvent *in vacuo* from the collected fractions yielded the 4'-(*N*-2-chloroethyl)amino-2,2':6',2"-terpyridine (797mg, 85%). Recrystallisation was achieved from diethyl ether/P.E. (40-60°C): m.p.135-137°C; (Found: C, 66.0; H, 4.5; N, 18.3. $C_{17}H_{15}N_4Cl$ requires C, 65.7; H, 4.8; N, 18.0); $\nu_{max}$(KBr)/$cm^{-1}$ 3256(m,br), 3134-2954(w,br) 1582(s), 1565(s), 1460(m), 1443(m), 1226(m), 985 (m), 793(s); $\delta_{1H}$ (500 MHz; $CDCl_3$) 8.68 (d, $^3J(6,5)$=4.7, 2H, H-C(6), H-C(6")); 8.62 (d, $^3J(3,4)$=7.97, 2H, H-C(3), H-C(3")); 7.84 (dt, $^3J(4,3)$=$^3J(4,5)$=7.7, $^4J(4,6)$=1.79, 2H, H-C(4), H-C(4")); 7.73 (s, 2H, H-C(3'), H-C(5')); 7.32 (ddd, $^3J(5,4)$=7.4, $^3J(5,6)$=4.8, $^4J(5,3)$=1.1, 2H, H-C(5), H-C(5")); 4.79 (t, $^3J_{vic}$=5.2, 1H, terpyN$\underline{H}$.$CH_2CH_2Cl$); 3.83-3.74 (m, 4H, terpyNH.$C\underline{H_2}C\underline{H_2}Cl$); m/z(ESI)=†311.18 (100%, $[MH]^+$).

**4'-Aziridino-2,2':6',2"-Terpyridine**

Sodium hydride (16mg, 0.6mmol) was washed with P.E. (40-60°C) and suspended in tetrahydrofuran (1ml). *t*-Butanol (38ml, 0.4mmol) was added and the mixture stirred for 5 min. until effervescence had subsided. After this time a solution of 4'-(*N*-2-chloroethyl)amino-2,2':6',2"-terpyridine (64mg, 0.2mmol) in THF (1ml) was introduced and reaction allowed to proceed at room temperature. 18 hrs later all solvent was removed *in vacuo* to yield a yellow solid. This solid was suspended in water (5ml) and the solution neutralised by the addition of a few drops of acetic acid. Organic material was extracted into dichloromethane (5ml), separated and dried over anhydrous magnesium sulphate, Filtration and evaporation to dryness yielded 4'-aziridino-2,2':6',2"-terpyridine as a white crystalline solid (54mg, 95%). Recrystallisation was achieved from acetonitrile: m.p. 149-151°C; (Found: C, 74.3; H, 4.7; N, 20.4. $C_{17}H_{14}N_4$ requires C, 74.4; H, 5.1; N, 20.4); $\nu_{max}$(KBr)/$cm^{-1}$ 3050-2000(s,br), 1800-1700(w,br), 1587(s), 1565(s), 1469(m), 1404(s), 1284 (m), 1160(m), 991(m), 880(m), 788(s); $\delta_H$ (200 MHz; $CDCl_3$) 8.73 (d, $^3J(6,5)$=4.1, 2H, H-C(6), H-C(6")); 8.64 (d, $^3J(3,4)$=7.89, 2H, H-C(3), H-C(3")); 8.12 (s, 2H, H-C(3'), H-C(5')); 7.87 (t, $^3J(4,3)$=$^3J(4,5)$=7.7, 2H, H-C(4), H-C(4")); 7.36 (ddd, $^3J(5,4)$=7.4, $^3J(5,6)$=4.8, $^4J(5,3)$=1.3, 2H, H-C(5), H-C(5")); 2.35 (s,4H, aziridine $C\underline{H_2}$'s); $\delta_{13C}$ (125.7 MHz; $CDCl_3$) 164.38 (C, C(4')); 156.41 (C, C(2), C(2"), C(2'), C(6')); 149.26 (CH, C(6), C(6")); 137.07 (CH, C(4), C(4")); 123.98 (CH, C(5), C(5")); 121.57 (CH, C(3), C(3")); 113.68 (CH, C(3'), C(5')); 27.66 ($CH_2$, aziridine C(1), C(2)); m/z(ESI)=275 (100%, $[MH]^+$).

Reaction has also been carried out using potassium *t*-butoxide directly 1.5ml of a solution of potassium *t*-butoxide (1.5g) in THF (10ml) was added to a solution of 4'-(*N*-2-chloroethyl)amino-2,2';6',2"-terpyridine (64mg, 0.2mmol) in THF (1ml). The mixture was stirred at room temperature for 21 hrs. Work up analogous to the above yielded 4'-aziridino-2,2':6',2"-terpyridine (50mg, 89%) with an NMR spectrum identical to the above.

**Synthesis of 4'(4-bromophenyl)-2,2':6,2"-terpyridine**

A mixture of 4-bromobenzaldehyde (7.40 g, 40 mmol), 2-acetylpyridine (10.0 g, 82 mmol), acetamide (35 g) and ammonium acetate (25 g) was heated in an oil bath at 180°C for 2 hr. The reaction mixture was cooled to 120°C and aqueous sodium hydroxide (18 g in 50 ml $H_2O$) was carefully added. The reaction was maintained at 120°C for another 2 hr. The aqueous phase was decanted while still hot. The residue was washed with water then dissolved in a small volume of warm glacial acetic acid. Concentrated HBr (48%, 100 ml) was added and the resulting suspension was kept in the fridge for 2-3 hr. The glossy precipitate of HBr salt was filtered by a sintered glass funnel and washed with 48% HBr then sucked to dryness. The solid was transferred to a flask with 50 ml of water. Aqueous sodium hydroxide (~ 5 M) was added dropwise with stirring until the solution is just basic to litmus (pH=8). The white suspension was extracted with dichloromethane (3x100 ml). The organic phase was dried ($MgSO_4$) and evaporated i*n vacuo* to give the crude product as a light brown residue (about 4 g). $^1$H nmr spectrum showed about 20% of contaminant which was removed as follows.

The residue from above (max. 4 g, 10 mmol) was heated to reflux with $FeCl_2.4H_2O$ in ethanol (50 ml) for 1 hr. The deep purple precipitate was centrifuged and washed several times with ether. The precipitate was dried and dissolved in aqueous acetonitrile 1:1. Aqueous sodium hydroxide (~5 M) was added dropwise until pH=10. The solution was then stirred vigorously under oxygen atmosphere until the purple colour was completely discharged (about 2 hr., more base may be added if necessary). The brown suspension was extracted with ether (5x100 ml). The ether layer was dried and evaporated *in vacuo*. The residue was recrystallised from ethanol to give the pure product as white fluffy needles (2.19 g): m.p. 139-141°C; $\delta_H$ (200 MHz. $CDCl_3$) 7.38 (dd J=6.1 J'=3.7 Hz, 2H. H4,4" or H5,5"), 7.66 and 7.81 (AB system J=8.6 Hz, 4H, H2.6 and H3,5-phenyl), 7.91 (dt J= 7.8 J'=1.7 Hz, 2H, H4,4" or H5,5"), 8.68-8.76 (m, 4H, overlapping H3,3" and H5,6"), 8.73 (s, 2H, H3,5'); m/z (In Beam EI) 387/389($M^+$, 100%), 308($[M-Br]^+$, 98), 229(32), 78 (100).

**4'-Fluoro-2,2':6',2"-terpyridine**

A suspension of 4'-amino-2,2':6',6"-terpyridine (0.185g ; 0.75mmol) in fluoroboric acid (50%; 1.0ml) was stirred and treated dropwise at 0°C with a cold solution of sodium nitrite (0.10g ; 1.4mmol) in water (0.5ml). Most of the solid dissolved to give a bright yellow solution. The mixture was stirred at 0°C for 1h and then allowed to warm up to room temperature and stirred for a further 1h. The mixture was cooled again to 0°C and basified with aqueous sodium hydroxide (50%). A yellow solid was precipitated and the mixture was extracted with chloroform (3 x 10ml). The organic extracts were dried and evaporated and the residual brown gum flash chromatographed over alumina eluting with light petroleum:ether (10:1). The fractions containing terpyridine were combined, evaporated and the material further purified by preparative tic on alumina to remove 4'-chloroterpyridine formed as a by-product (7%). Elution six times with light petroleum:ether (50:1) afforded the title compound as a colourless solid. (0.043g ; 23%). Crystallisation from light petroleum-dichloromethane gave colourless needles mp 114-115°C (Found: C, 71.4; H, 4.0; N, 16.5. $C_{15}H_{10}FN_3$ requires C, 71.7; H, 4.0; N, 16,7%); $\upsilon_{max}$(nujol)/$cm^{-1}$ 1597, 1581, 1468, 1403, 1173, 931 and 790; $\delta_H$ (200MHz $CDCl_3$) 7.36 (2H, ddd, J 0.9, 4.8 and 7.4, terpyH5,5"),7.86 (2H, td, J 1.8, 7.7, terpy H4,4"), 8.19 (2H, d, $J_{HF}$ 9.7, terpyH3',5'), 8.61 (2H, d, J 8.6, terpyH3,3") and 8.70 (2H, d, J 4.5, terpyH6,6"); δC(50MHz CDCl3) 108.5 (d, $J_{CF}$ 19.5, C3',5'), 121.3, 124.3, 136.9 (C3,3"), 149.2 (C6.6"), 155.0 (C2,2"), 158.6 (d, $J_{CF}$ 7.3, C2',6') and 170.77 (d, $J_{CF}$ 259.8, C4'); $\delta_F$(235MHz $CDCl_3$) -102.0; m/z($APCl^+$) 252($MH^+$).

**4'-Azido-2,2':6',2"-terpyridine**

A solution of 4'-hydrazino-2,2':6',6"-terpyridine (0.263g ; 1.0mmol) in acetic acid (2.0ml) and water (1.0ml) was treated dropwise at 0°C with a cold solution of sodium nitrite (0.69g ; 10mmol) in water (2.0ml). A pale brown solid was precipitated. When the addition was complete, ether (20ml) was added and the aqueous layer made alkaline by the addition of solid sodium hydroxide beads. The solid dissolved and the aqueous phase was further extracted with ether (2 x 20ml). The combined organic extracts were dried and evaporated to give the title compound as a pale yellow brown solid (0.273g ; 99%). Crystallisation from methanoldichloromethane gave pale yellow needles mp. 140-141°C (Found: C, 65.7; H, 3.6; N, 30.5. $C_{15}H_{10}N_3$ requires C, 65.7; H, 3.7; N, 30.6%); $\nu_{max}$(nujol)/$cm^{-1}$ 2109 (N3); $\delta_H$(200MHz $CDCl_3$) 7.37 (2H, ddd, *J* 1.0, 4.8 and 5.9, terpy H5,5"),7.87 (2H, td, *J* 1.8, 7.8, terpy H4,4"), 8.16 (2H, s, terpyH3',5'), 8.63 (2H, d, *J* 8.0, terpyH3,3") and 8.68-8.74 (2H, m, terpyH6,6"); $\delta_c$(50MHz $CDCl_3$) 111.1, 121.3, 124.1, 136.8, 149.1, 150.8, 155.2, 157.1; m/z(ESI) 275($MH^+$).

**4'-Amino-2,2':6',2"-terpyridine**

A solution of 4'-azido-2,2':6',6"-terpyridine (0.453g ; 1.65mmol) in dichloromethane (5,0ml) and methanol (5.0ml) was saturated with hydrogen sulphide at 0°C and the solution kept at room temperature for 4h by which time tic showed no azide to remain. The solution was degassed with argon to remove hydrogen sulphide and then evaporated to dryness. The residue was treated with sulphuric acid (2M, 3.0ml) and the aqueous mixture filtered and washed with dichloromethane to remove elemental sulphur. The solution was basified by the dropwise addition of sodium hydroxide (50%) and extracted with dichloromethane (3 x 20ml). The combined organic layers were dried and evaporated to give the title compound as a pale yellow solid.(0.398g ; 97%). Crystallisation from light petroleum-dichloromethane gave pale yellow brown needles mp. 228-30°C (Found: C, 72.5; H, 4.7; N, 22.2. $C_{15}H_{12}N_4$ requires C, 72.6; H, 4.9; N, 22.5%); $\nu_{max}$(nujol)/$cm^{-1}$ 3414, 3344 and 3226 ($NH_2$); $\delta_H$(200MHz $CDCl_3$) 4.36 (2H, bs, $NH_2$), 7.32 (2H, ddd, *J* 1.2, 4.8 and 6.0, terpy H5,5"), 7.75 (2H, s, terpyH3',5'), 7.84 (2H, td, *J* 1.8, 7.7, terpy H4,4"), 8.60 (2H, d, *J* 8.0, terpyH3,3"), and 8.66-8.69 (2H, m, terpyH6,6"); m/z(ESI) 249($MH^+$).

**(4-Picoline)(4'-Hydrazino-2,2':6',2''-Terpyridine) Platinum (II) Tetrafluoroborate (R)**

The general procedure for platination was carried out using methanol as the initial solvent and 4'-hydrazinoterpyridine (25mg, 0.1mmol). Recrystallisation from acetonitrile gave the title compound as yellow-green crystals (49mg, 71%): m.p >200°C; (Found: C, 35.1; H, 2.3; N, 11.7. $PtC_{21}H_{20}N_6B_2F_8$ requires C, 34.8; H, 2.7; N, 11.6); $\nu_{max}$(KBr)/cm$^{-1}$ 3333(s,br) 3089(m,br), 1622(s), 1481(m), 1150-900(s, br), 786(m); $d_{1H}$ (500 MHz; $CD_3CN$) 8.82 (dd with broad $^1$H-195P† satellites, $^3J$(2,3)= 5.2, $^5J$(2,5)= 1.4, 2H, picoline H-C(2), H-C(6)); 8.34 (dt, $^3J$(4,3)=$^3J$(4,5)=7.8, $^5J$(4,6)=1.45, 2H, H-C(4), H-C(4'')); 8.30-8.18 (s, br, 2H, H-C(3), H-C(3'')); 7.94 (s, br, 2H, H-C(3'), H-C(5')); 7.72 (dd, 3J(3,2)=4.6, $^4J$(3,6)= 0.9, 2H, picoline H-C(3), H-C(5)); 7.70 (d, $^3J$(6,5)=5.5, 2H, H-C(6), H-C(6'')); 7.62 (ddd, $^3J$(5,4)=7.6, $^3J$(5,6)=5.7, 2H, H-C(5), H-C(5'')); 7.45-7.30 (s, br, 1H, terpyN$\underline{H}$.$NH_2$); 4.40 (s, 2H, terpyNH.N$\underline{H}_2$); 2.63 (s, 3H, picoline CH3)); m/z (ESI)= 275.76 (95%, $[M]^{2+}$).

**(4-Picoline)(4'-(1-Methylhydrazino)-2,2':6',2''-Terpyridine) Platinum (II) Tetra-fluoroborate (S)**

The general procedure for platination was carried out using methanol as the initial solvent and 4'-(1-methylhydrazino)terpyridine (23mg, 0.084mmol). Recrystallisation from acetonitrile gave the title compound as orange crystals (51mg, 83%): m.p >200°C; (Found: C, 36.7, H, 2.7; N, 12.3. $PtC_{22}H_{22}N_6B_2F_8.CH_3CN$ requires C, 36.9; H, 3.2; N, 12.5); $\nu_{max}$(KBr)/cm$^{-1}$ 3359(m), 3093(m,br), 2251(w), 1646-1609(s,br), 1212(m), 1150-950(s,br), 785(s); $\delta_{1H}$ (500 MHz; $CD_3CN$)* 8.82 (dd with broad $^1$H-$^{195}$Pt satellites, $^3J$(2,3)=5.2, $^5J$(2,5)=1.4, 2H, picoline H-C(2), H-C(6)); 8.35-8.15 (m, br, 4H, H-C(3), H-C(3''), H-C(4), H-C(4''); 7.73 (dd, $^3J$(3,2)=5.6, $^5J$(3,6)=1.00, 2H, picoline H-C(3), H-C(5)); 7.70 (d, $^3J$(6,5)=6.6, 2H, H-C(6), H-C(6'')); 7.62 (ddd, $^3J$(5,4)=7.3, $^3J$(5,6)=6.0, $^4J$(5,3)=2.0, 2H, H-C(5), H-C(5'')); 4.71 (s, 2H, terpyNMe.N$\underline{H}_2$); 3.55 (s, 3H, terpyNC$\underline{H}_3$$NH_2$); 2.63 (s, 3H, picoline C$\underline{H}_3$); m/z (ESI)= 282.8(95%, $[M]^{2+}$). *H-C(3'), H-C(5') resonance not observed at high temperature or by COSY.

**(4-Picoline)(4'-(*N*-2-Hydroxyethyl)amino-2,2':6',2''-Terpyridine) Platinum (II) Tetrafluoroborate (U)**

The general procedure for platination was carried out using acetone as the initial solvent and 4'-aziridinoterpyridine (24mg, 0.086mmol), Recrystallisation from acetonitrile gave the title compound as orange crystals (41mg, 63%): m.p >200°C; (Found: C, 35.7; H, 2.8; N, 9.2. $PtC_{23}H_{23}N_5OB_2F_8.H_2O$ requires C, 35.7; H, 3.2; N, 9.1); $\nu_{max}$(KBr)/cm$^{-1}$ 3349(m,br), 3103(m,br), 1626(s,br), 1482(s), 1360(m), 1213-1083(s,br), 786(s); $\delta_{1H}$ (200 MHz; $CD_3CN$) 8.81 (d, br with broad $^1$H-$^{195}$Pt satellites, 2H, picoline H-C(2), H-C(6)); 8.38-8.20 (m, br, 4H, H-C(3), H-C(3''), H-C(4), H-C(4'')); 7.80-7.68 (m, br, 4H, H-C(5), H-C(5''), picoline H-C(3), H-C(5)); 7.60 (s, br, 2H, H-C(6), H-C(6'')); 7.40 (s, br, 2H, H-C(3'), H-C(5')); 6.90 (s, br, 1H, terpyN$\underline{H}$.$CH_2CH_2OH$); 3.78 (t, $^3J_{vic}$=5.0, 2H, terpyNH.$CH_2$C$\underline{H}_2$OH); 3.58 (q, $^3J_{vic}$=5.1, 2H, terpyNH.C$\underline{H}_2$$CH_2OH$); 3.30-3.20 (s, br, 1H, terpyNH.$CH_2CH_2$O$\underline{H}$); 2.62 (s, 3H, picoline C$\underline{H}_3$); m/z (ESI)= 290.33 (95%, $[M]^{2+}$).

**Evaluation of the Stability of (4-Picoline)(4'-Aziridino-2,2':6',2''-Terpyridine) Platinum (II) Tetrafluoroborate in Aqueous Solution:-**

A solution of (4-picoline) (4'-aziridinoterpyridine) platinum (II) tetrafluoroborate in deuterated water was prepared. NMR spectra were recorded immediately, after 1, 2 and 17 hours, then after one and two weeks. No change in the spectra was observed indicating the compound to be stable to nucleophilic attack by water under neutral conditions.

**Evaluation of the Acid/Base Stability of 4'-Aziridino-2,2':6',2''-Terpyridine**

Diethylamine (2ml) was added to a solution of 4'-aziridinoterpyridine (5mg) in deuterated chloroform in a NMR tube. No change in the NMR spectrum (other than the appearance of resonances attributed to diethylamine) was observed directly afterwards or after 20 hours.

Trifluoroacetic acid (5µl) was added to a solution of 4'-aziridinoterpyridine (15mg) in deuterated methanol in an NMR tube. NMR spectra were recorded immediately and after 1, 2, 4 and 24 hours. All spectra showed new signals in both the aromatic and aliphatic regions in addition to those due to the starting material, indicating the aziridine to be susceptible to nucleophilic attack in the presence of $H^+$.

**(4-Picoline)(4'-Aziridino-2,2':6',2''-Terpyridine) Platinum (II) Tetrafluoroborate (T)**

The general procedure for platination was carried out utilising acetonitrile as the initial solvent. The platinum (II) containing supernatant was added to a solution of 4'-aziridinoterpyridine (33mg, 0.12mmol) and 1,3-diaminopropane (5ml, 0.06mmol), again in MeCN. No acetonitrile complex could be isolated. After reaction with 4-picoline a solid precipitated below a supernatant. NMR of this solid showed it to be void of the terpyridine moiety and therefore it is probably more AgI. Addition of diethyl ether to the supernatant yielded a tarry solid which became crystalline on trituration with further $Et_2O$. These crystals were collected by filtration and recrystallised from MeCN by diffusion of $Et_2O$ to yield the title compound (56mg, 82%): m.p >200°C; (Found: C, 36.6; H, 3.2; N, 9.4. $PtC_{23}H_{21}N_5B_2F_8.H_2O$ requires C, 36.6; H, 3.1; N, 9.3); $\nu_{max}$(KBr)/cm$^{-1}$ 3500-3200(w,br), 3080(w,br), 1610(m), 1480(m), 1436(m), 1298(w), 1058(s,br), 792(m); $\delta_{1H}$ (500 MHz; $CD_3CN$) 8.81 (dd, br with broad $^1$H-$^{195}$Pt satellites, $^3J$(2,3)=5.3, $^5J$(2,5)=1.3, 2H, picoline H-C(2), H-C(6)); 8.40 (dt, $^3J$(4,3)=7.89, $^4J$(4,5)=1.59, 2H, H-C(4), H-C(4")); 8.31 (d, br, $^3J$(3,4)= 7.86, 2H, H-C(3), H-C(3")); 7.97 (s, 2H, H-C(3'), H-C(5')); 7.75 (dd, $^3J$(3,2)=5.6, $^5J$(3,6)=1.2, 2H, picoline H-C(3), H-C(5)); 7.71 (d, br, $^3J$(6,5)= 6.29, 2H, H-C(6), H-C(6")); 7.68 (ddd, $^3J$(5,6)=5.87, $^4J$(5,3)=1.6, 2H, H-C(5), H-C(5")); 2.64 (s, 3H, picoline C$\underline{H}_3$); 2.61 (s, 4H, aziridine C$\underline{H}_2$'s); m/z (ESI)=281.3(100%, $[M]^{2+}$).

**Adipoyl-Linked Bis[4'-Hydrazino-2,2':6',2"-Terpyridine]**

Adipoyl chloride (175µl, 1.2mmol) was added to a solution of 4'-hyrazinoterpyridine (178mg, 2.2mmol) in 10ml of tetrahydrofuran. Immediately the solution turned yellow and a yellow solid became suspended in solution. After stirring at room temperature for 2 hours dichloromethane (100ml) and saturated aq. sodium bicarbonate solution (100ml) were added. The yellow solid turned white and collected at the solvent interface. Isolation of this solid by filtration, and washing with diethyl ether yielded the titled product (387mg, 55%). A sample for elemental analysis was prepared as the acetate salt: m.p >200°C; (Found C, 63.8; H, 5.2. $C_{36}H_{32}N_{10}O_2.C_4H_8O_4$ requires C, 63.5 H, 5.3). $\nu_{max}$ (KBr)/cm$^{-1}$ 3283-3012(s,br), 2928-2800(w,br), 1655(s), 1585(s), 1566(s), 1467(s), 1405(s), 989(m), 866(w), 793(s), 745(m), 733(m); $\delta_{1H}$ (500MHz; DMSO) 9.99 and 8.80 (2s, br, 2x2H, [terpyN$\underline{H}$.N$\underline{H}$COCH$_2$CH$_2$]$_2$); 8.68 (d, $^3J$(6,5)= 4.75, 4H, H-C(6), H-C(6")); 8.57 (d, $^3J$(3,4)=7.91, 4H, H-C(3), H-C(3")); 7.95 (dt, $^3J$(4,5)=$^3J$(4,3)=7.76, $^4J$(4,6)=1.80, 4H, H-C(4), H-C(4")); 7.80 (s, 4H, H-C(3'), H-C(5')); 7.44 (dd, $^3J$(5,4)=7.48, $^3J$(5,6)=4.73, 4H, H-C(5), H-C(5")); 2.34 (m, br, [terpyNH.NHCOC$\underline{H}_2$CH$_2$]$_2$); 1.74 (m, br, [terpyNH.NHCOCH$_2$C$\underline{H}_2$]$_2$); $\delta_{13C}$ (125.7MHz: DMSO) 172.07 (C, [terpyNH.NH$\underline{C}$OCH$_2$CH$_2$]$_2$); 157.05 (C, C(4')); 155.76 (C, C(2'), C(6') or C(2), C(2")); 155.34 (C, C(2'), C(6') or C(2), C(2")); 149.21 (CH, C(6), C(6")); 137.22 (CH, C(4), C(4")); 124.14 (CH, C(5), C(5")); 120.79 (CH, C(3), C(3")); 103.67 (CH, C(3'), C(5')); 33.27 (CH2, [terpyNH.NHCO$\underline{C}$H$_2$CH$_2$]$_2$); 24.96 (CH$_2$, [terpyNH.NHCOCH$_2\underline{C}$H$_2$]$_2$); m/z (ESI)=637(<10%, $[MH]^+$), 319 (100%, $[M+2H]^{2+}$).

**Adipoyl-Linked Bis[4'-(1-Methylhydrazino)-2,2':6',2"-Terpyridine]**

This was synthesised by the addition of adipoyl chloride (430µl, 3mmol) to a solution of 4'-(1-methylhydrazino) terpyridine (1.49g, 5.4mmol) in tetrahydrofuran (20ml). A white solid at the solvent interface was collected by filtration and washed with diethyl ether to give the title product (90mg, 50%): m.p.>200°C; $\nu_{max}$(KBr)/cm$^{-1}$3291(s,br), 3064(w, br), 2900-2800(m,br), 1657(s), 1585(s), 1564(s), 1511(s), 1467(s), 1283(m), 1111(m), 1007(m), 854(m), 791(s), 731 (m); $\delta_{1H}$ (500MHz; DMSO) 10.52 (s, 2H, [terpyNMe.N$\underline{H}$COCH$_2$CH$_2$]$_2$); 8.70 (d, $^3J$(6,5)=4.69, 4H, H-C(6), H-C(6")); 8.60 (d, $^3J$ (3,4)=7.92, 4H, H-C(3), H-C(3")); 7.97 (dt, $^3J$ (4,5)=$^3J$(4,3)=7.72. $^4J$(4,6)=1.79, 4H, H-C(4), H-C(4")); 7.81 (s, 4H, H-C(3'), H-C(5')); 7,45 (ddd, $^3J$(5,4)=7.4, $^3J$ (5,6)=4.80, $^4J$(5,3)=1.16, 4H, H-C(5), H-C(5")); 3.30 (s, 6H, [terpyNC$\underline{H}_3$.NHCOCH$_2$CH$_2$]$_2$); 2.33 (s, br, 4H [terpyNMe.NHCOC$\underline{H}_2$CH$_2$]$_2$); 1.75 (s, br, 4H [terpyNMe.NHCOCH$_2$C$\underline{H}_2$]$_2$); $\delta_{13C}$ (125.7MHz; DMSO)* 171.41 (C, [terpyNMe.NH$\underline{C}$OCH$_2$CH$_2$]$_2$); 156.75 (C, C(4')); 155.70 (C, C(2'), C(6') or C(2), C(2")); 155.47 (C, C(2'), C(6') or C(2), C(2")); 149.24 (CH, C(6), C(6")); 137.31 (CH, C(4), C(4")); 124.26 (CH, C(5), C(5")); 120.94 (CH, C(3), C(3")); 103.43 (CH, C(3'), C(5')); 33.17 (CH$_2$, [terpyNMe.NHCO$\underline{C}$H$_2$CH$_2$]$_2$); 24.80 (CH$_2$, [terpyNMe.NHCOCH$_2\underline{C}$H$_2$]$_2$) m/z(ESI)= 665 (<10%, $[MH]^+$), 333 (100%, $[M+2H]^{2+}$). *A signal due to the primary C atom [terpyN$\underline{C}$H$_3$.NHCOCH$_2$CH$_2$]$_2$ was not observed, probably due to it being obscured by the DMSO solvent resonance.

**Adipoyl-Linked-Bis[4-Picoline(4'-Hydrazino-2,2':6',2"-Terpyridine) Platinum(II)] Tetrafluoroborate (V)**

The general procedure for platination was carried out using acetone as the initial solvent using adipoyl-linked bis(4'-hyrazinoterpyridine) (20mg, 0.03mmol), diiodo(1,5-cyclooctadiene)platinum(II) (84mg, 0.15mmol) and silver tetrafluoroborate (80mg, 0.32mmol). Vigorous stirring and long reaction times (60 minutes) were required owing to the poor solubility of the terpyridine in acetonitrile. Recrystallisation from MeCN and diethyl ether gave yellow crystals of the title product (33mg, 67%): m.p.>200°C; (Found: C, 36.6; H, 3.4; N, 11.1; $Pt_2C_{48}H_{46}N_{12}O_2B_4F_{16}$ requires C, 36.9; H, 3.0 N, 10.8 $Pt_2C_{48}H_{46}N_{12}O_2B_4F_{16}$ .$H_2O$ requires C, 36.5; H, 3.1; N, 10.7); $\nu_{max}$(KBr)/cm$^{-1}$ 3330-3200(s,br), 3087 (m,br), 2360(w), 1690(m), 1625(s), 1483(m), 1100-1000(s,br), 788(m); $\delta_{1H}$ (500MHz; $CD_3CN$)* 8.77 (s, br, [Pt$^{2+}$pic. terpyN$\underline{H}$.NHCOCH$_2$CH$_2$]$_2$ or [Pt$^{2+}$pic. terpyNH.N$\underline{H}$COCH$_2$CH$_2$]$_2$); 8.74 (d, br, picoline H-C(2), H-C(6)); 8.34-8.17 (m, br. H-C(3), H-C(3"), H-C(4), H-C(4")); 7.60 (d, br, picoline H-C(3), H-C(5)); 7.55 (s, br, H-C(5), H-C(5")); 2.63 (s, picoline C$\underline{H}_3$); 2.54 (m, br, [Pt$^{2+}$pic.terpyNH. NHCOC$\underline{H}_2$CH$_2$]$_2$); 1.87 (m, br, [Pt$^{2+}$pic.terpyNH.NHCOCH$_2$C$\underline{H}_2$]$_2$).

**Adipoyl-Linked-Bis[4-Picoline(4'-(1-Methylhydrazino)-2,2':6',2''-Terpyridine) Platinum(II)] Tetrafluoroborate (W)**

This was synthesised according to the procedure used for compound (V). Recrystallisation from acetonitrile and diethyl ether gave yellow crystals of the title product (36mg, 75%): m.p.>200°C; (Found: C, 37.3; H, 3.2; N, 10.3; $Pt_2C_{50}H_{50}N_{12}O_2B_4F_{16}.H_2O$ requires C, 37.4; H, 3.2; N, 10.4); $\nu_{max}$(KBr)/cm$^{-1}$ 3325(w,br), 3086(w,br), 1686 (m), 1622 (s), 1554(w), 1484(m), 1255(w), 1100-1000(s,br), 789(m), 523(w); $\delta_{1H}$ (500MHz; $CD_3CN$) 8.97 (s, br, [$Pt^{2+}$pic.terpyN-Me.N$\underline{H}$CO$CH_2CH_2$]$_2$); 8.77 (d, 4H, picoline H-C(2), H-C(6)); 8.47-8.36 (m, br, 8H, H-C(3), H-C(3''), H-C(4), H-C(4'')); 7.71 (d, br, 8H, H-C(6), H-C(6''), picoline H-C(3), H-C(5)); 7.65-7.62 (m, br, 4H, H-C(5), H-C(5'')); 3.43 (s, 6H, [$Pt^{2+}$pic. terpyNC$\underline{H}_3$.NHCO$CH_2CH_2$]$_2$); 2.64 (s, 6H, picoline C$\underline{H}_3$); 2.52 (s, br, 4H, [$Pt^{2+}$pic.terpyNMe.NHCOC$\underline{H}_2$$CH_2$]$_2$); 1.86 (s, br, 4H, [$Pt^{2+}$pic.terpyNMe.NHCO $CH_2$C$\underline{H}_2$]$_2$); m/z(ESI)= 310.3 (100%, $[M]^{4+}$).

**4,4'-Vinylidenedipyridine bis[2,2':6',2''-terpyridine platinum II] tetrafluoroborate ($A_{14}$)**

Cyclooctadienylplatinum II diiodide (0.292g ; 0.53mmol) was treated with a solution of silver tetrafluoroborate (0.214g ; 1.1mmol) in acetone (1.5ml). The mixture was centrifuged to remove precipitated silver iodide and the supernatant solution added to a solution of 2,2:6',2''-terpyridine (0.117g ; 0.5mmol) in dichloromethane (0.25ml) and acetonitrile (0.25ml). A yellow orange solid was immediately precipitated and was collected by centrifugation and washed with ether:acetonitrile (2:1) (2 x 1.0ml). The solid was then suspended in acetonitrile (0.5ml) and the mixture treated with a solution of 4,4'-vinylidenedipyridine (0.046g ; 0,25mmol) in dichloromethane (0,25ml). The mixture was kept at room temperature with occasional shaking for 5h. The solid changed to a paler yellow colour The product was collected by centrifugation and dried over $P_2O_5$ in vacuo to give the title compound (0.123g, 35%). mp. >230°C

**Aquo 4'-chloro-2,2':6',2''-terpyridine platinum II tetrafluoroborate ($I_{11}$)**

Cyclooctadienylplatinum II diiodide (0.292g ; 0.53mmol) was treated with a solution of silver tetrafluoroborate (0.214g ; 1.1mmol) in acetone (1.5ml). The mixture was centrifuged to remove precipitated silver iodide and the supernatant solution added to a 2suspension of 4'-chloro-2,2:6',2''-terpyridine (0.133g ; 0.5mmol) in dichloromethane (0.5ml) and ether (0.25ml). The mixture turned yellow and a yellow solid was precipitated. This was collected by centrifugation and washed with ether (2 x 1.0ml). The solid was allowed to dry in the air and was then suspended in water (0.5ml). The mixture was sonicated in a water bath for 5min and then the supernatant liquor removed. Further water (1.0ml) was added and sonication continued for a further 5min. The resulting yellow solid was collected by centrifugation and dried over $P_2O_5$ *in vacuo* to give the title compound.

**Ammonio 4'-chloro-2,2':6',2''-terpyridine platinum II tetrafluoroborate ($I_{12}$)**

Cyclooctadienylplatinum II diiodide (0.292g ; 0.53mmol) was treated with a solution of silver tetrafluoroborate (0214g ; 1.1mmol) in acetone (1.0ml). The mixture was centrifuged to remove precipitated silver iodide and the supernatant solution added to a suspension of 4'-chloro-2,2:6',2''-terpyridine (0,133g ; 0.5mmol) in dichloromethane (0.5ml). The mixture turned yellow and a yellow solid was precipitated. This was collected by centrifugation and washed with acetone : ether (1:1) (1.0ml), ether (1.0ml) and finally ether saturated with ammonia (2 x 1.0ml) to form the amine complex. This afforded the title compound as a pale yellow solid (0.110g. 34%).

**4,4'-Vinylidenedipyridine bis[4'-chloro-2,2':6',2''-terpyridine platinum II]tetrafluoroborate ($I_{14}$)**

Cyclooctadienylplatinum II diiodide (0.292g ; 0.53mmol) was treated with a solution of silver tetrafluoroborate (0.214g ; 1.1mmol) in acetone (1.5ml). The mixture was centrifuged to remove precipitated silver iodide and the supernatant solution added to a suspension of 4'-chloro-2,2:6',2''-terpyridine (0.134g ; 0.5mmol) in dichloromethane (0.5ml). An off-white solid was precipitated and was collected by centrifugation and washed with ether:acetonitrile (2:1) (2 x 1.0ml). The solid was then suspended in acetonitrile (0.5ml) and the mixture treated with a solution of 4,4'-vinylidenedipyridine (0.046g ; 0.25mmol) in dichloromethane (0.25ml). The mixture was kept at room temperature with occasional shaking for 18h. The resulting cream solid was collected by centrifugation washed with ether and dried over $P_2O_5$ in vacuo to give the title compound (0.107g, 30%). mp. >230°C

**Aquo 4'-(4-bromophenyl)-2,2':6',2''-terpyridine platinum II tetrafluoroborate ($Q_{11}$)**

Cyclooctadienylplatinum II diiodide (0.292g ; 0.53mmol) was treated with a solution of silver tetrafluoroborate (0.214g; 1.1mmol) in acetone (1.5ml). The mixture was centrifuged to remove precipitated silver iodide and the supernatant solution added to a suspension of 4'-(4-bromophenyl)-2,2:6',2"-terpyridine (0.194g ; 0.5mmol) in dichloromethane (0.5ml) and ether (0.5ml). The mixture turned orange and then lightened to yellow and a yellow solid was precipitated. This was collected by centrifugation and washed with ether (2 x 1.0ml). The solid was allowed to dry in the air and was then suspended in water (0.5ml). The mixture was sonicated in a water bath for 5min and then the supernatant liquor removed. Further water (1.0ml) was added and sonication continued for a further 5min. The resulting yellow solid was collected by centrifugation and dried over $P_2O_5$ *in vacuo* to give the title compound.

**Ammonio 4'-(4-bromophenyl)-2,2':6',2"-terpyridine platinum II tetrafluoroborate ($Q_{12}$)**

Cyclooctadienylplatinum II diiodide (0.292g ; 0.53mmol) was treated with a solution of silver tetrafluoroborate (0214g ; 1.1mmol) in acetone (1.0ml). The mixture was centrifuged to remove precipitated silver iodide and the supernatant solution added to a suspension of 4'-(4-bromophenyl)-2,2:6',2"-terpyridine (0.194g ; 0.5mmol) in dichloromethane (0.5ml). The mixture turned orange and a gum was precipitated. On rubbing this triturated to give a yellow solid which was collected by centrifugation and washed with acetone (1.0 ml), acetone : ether (1:1) (1.0ml), ether (1.0ml) and finally ether saturated with ammonia (2 x 1.0ml) to form the amine complex. This afforded the title compound as a yellow solid (0.265g, 68%).

**4,4'-Vinylidenedipyridine bis[4'-(4-bromophenyl)-2,2':6',2"-terpyridine platinum II]tetrafluoroborate ($Q_{14}$)**

Cyclooctadienylplatinum II diiodide (0.292g ; 0.53mmol) was treated with a solution of silver tetrafluoroborate (0.214g ; 1.1mmol) in acetone (1.5ml). The mixture was centrifuged to remove precipitated silver iodide and the supernatant solution added to a suspension of 4'-(4-bromophenyl)-2,2:6',2"-terpyridine (0,194g ; 0.5mmol) in dichloromethane (0.5ml). The mixture turned orange and a gummy red solid was precipitated. Acetonitrile (025ml) was added and on rubbing the red gum triturated to give a bright yellow solid. This was collected by centrifugation and washed with ethe:acetonitrile (2:1) (2 x 1.0ml). The solid was then suspended in acetonitrile (0.5ml) and the mixture treated with a solution of 4,4'-vinylidenedipyridine (0.046g 0.25mmol) in dichloromethane (0,25ml), The mixture was kept at room temperature with occasional shaking for 16h. The resulting yellow solid was collected by centrifugation and dried over $P_2O_5$ in vacuo to give the title compound (0.395g, 93%). mp. >230°C

**4-Picoline 4'-amino-2,2':6',2"-terpyridine platinum II tetrafluoroborate (X)**

Cyclooctadienylplatinum II diiodide (0,184g ; 0.33mmol) was treated with a solution of silver tetrafluoroborate (0.128g ; 0.66mmol) in acetone (1.0ml). The mixture was centrifuged to remove precipitated silver iodide and the supernatant solution added to a solution of 4'-amino-2,2:6',2"-terpyridine (0.074g ; 0.30mmol) in dichloromethane (2.0ml) and acetone (1.0ml). The mixture turned bright yellow and a yellow orange solid was precipitated. This was collected by filtration and washed with acetone. The solid was suspended in acetonitrile (3.0ml) and treated with 4-picoline (44ml ; 0.45mmol). The solid dissolved after stirring for 30min and the orange red solution was then added dropwise to ether (20ml). The yellow orange solid precipitated was filtered off and washed with ether and dried in vacuo to give the title compound (0.128g ; 60%).mp >230°C decomp.(Found: C, 35.2; H, 2.65; N, 9.7. $C_{21}H_{19}B_2F_8N_5Pt$ requires C, 35.5; H, 2.7; N, 9.8%); $\nu_{max}$(nujol)/$cm^{-1}$ 3428, 3352 and 3245 (NH2); $\delta_H$(200MHz CD3CN) 2.61 (3H, s, pyMe), 6.47 (2H. s, NH2) 7.41 (2H, s, terpyH3',5'), 7.50-7.79 (6H, m, pyH3,5 and terpyH4',4" and 5,5"), 8.04-8.37 (4H, m, pyH2,6 and terpyH3'3"),8.65-9.02 (2H, m, terpy H6.6"): m/z(ESI) 268.3($M^{2+}$).

**4-Picoline 4'-azido-2,2':6',2"-terpyridine platinum II tetrafluoroborate (Z)**

Cyclooctadienylplatinum II diiodide (0.060g ; 0.11mmol) was treated with a solution of silver tetrafluoroborate (0.043g ; 0.2mmol) in acetone (0.5ml). The mixture was centrifuged to remove precipitated silver iodide and the supernatant solution added to a solution of 4'-azido-2,2:6',2"-terpyridine (0.0278 ; 0.10mmol) in dichloromethane (0.5ml) and acetonitrile (0.25ml). The mixture turned yellow and a yellow solid was precipitated. This was collected by centrifugation and washed with acetone (1.0ml). The solid was suspended in acetonitrile and treated with " 4-picoline (50ml ; 0.51mmol). The solid dissolved and the brown solution was kept at room temperature for 16h before being added dropwise to ether (7.5ml). The title compound was precipitated as a pale brown powder (0.061g ;82%) collected by centrifugation and washed with ether (2 x 1.0ml). mp >230°C (Found: C, 34.2; H, 2.4; N, 13.1. $C_{21}H_{17}B_2F_8N_7Pt$ requires C, 34.2; H, 2.3 N, 13.3%); $\nu_{max}$(nujol)/$cm^{-1}$ 2123 ($N_3$); $\delta_H$(200MHz $CD_3CN$) 2.64 (3H, s, pyMe), 7.69-7.79 (6H, m, pyH3,5 and terpyH4',4" and 5,5"), 8.00 (2H, s, terpyH3',5') 8.33-8.48 (4H, m, pyH2,6 and terpyH3'3"),8.72-8.86 (2H, m, terpy H6,6"); m/z(ESI) 281($M^{2+}$).

Table 1

| **The 96 hour $IC_{50}$ values (in μM) for the *in vitro* growth inhibition of human ovarian cell lines**<br>Two of the cell lines are resistant to cisplatin and one to doxorubicin. RF is the resistance factor: $IC_{50}$ resistant line/ $IC_{50}$ parent line | | | | | | | | | |
|---|---|---|---|---|---|---|---|---|---|
| Compound | CH1 | $CH1cis^R$ | RF | $CH1dox^R$ | RF | A2780 | $A2780cis^R$ | RF | SKOV3 |
| cisplatin | 0.4 | 1.2 | 3.0 | | | 0.53 | 8.8 | 16.6 | 2.25 |
| A | $14^v$ | $11^v$ | 0.8 | 4.4 | 0.3 | $17^v$ | $>100^v$ | - | $20^v$ |
| $A_1$ | 2.1 | 2.1 | 1.0 | 0.85 | 0.41 | 5.8 | 6.7 | 1.16 | 9.2 |
| $A_2$ | 2.2 | 2.3 | 1.05 | 1.18 | 0.54 | 5.9 | 7.5 | 1.25 | 11.1 |
| $A_3$ | 6.1 | 7.5 | 1.2 | 3.95 | 0.6 | 16 | 21.5 | 1.3 | 13 |
| $A_4$ | 15.5 | 14 | 0.9 | 5.4 | 0.3 | 21.5 | >100 | - | >100 |
| $A_5$ | 17 | 17 | 1 | 6.1 | 0.3 | 19.5 | >100 | - | >100 |
| $A_6$ | 17 | 17 | 1 | 6.3 | 0.4 | 18.6 | >100 | 1 | >100 |
| $A_7$ | 16.5 | 15.5 | 0.9 | 6.1 | 0.4 | 17 | >100 | - | >100 |
| $A_8$ | 18 | 18.5 | 1 | 13 | 0.7 | 50 | 100 | 2 | >100 |
| $A_9$ | 14 | 12.5 | 0.9 | 5 | 0.3 | 40 | 92 | 2.3 | 17 |
| $A_{10}$ | 17.5 | 18 | 1 | 5.7 | 0.3 | 40 | >100 | - | 24 |
| $A_{11}$ | 12 | 18 | 1.5 | 14 | 1.2 | 8.8 | 7.0 | 0.9 | 9.2 |
| $A_{12}$ | 5.2 | 4.5 | 0.9 | 3.75 | 0.7 | 11.5 | 23.5 | 2 | 15 |
| $A_{13}$ | 6.6 | 6.4 | 1 | 3.75 | 0.6 | 49 | 41 | 0.8 | 19.5 |
| $A_{14}$ | 1.35 | 0.63 | 0.46 | 5.1 | 3.8 | 1.6 | 2.4 | 1.5 | 1.3 |
| v = variable | | | | | | | | | |

Table 2

| **The 96 hour $IC_{50}$ values (in μM) for the *in vitro* growth inhibition of human ovarian cell lines**<br>Two of the cell lines are resistant to cisplatin and one to doxorubicin. RF is the resistance factor: $IC_{50}$ resistant line/ $IC_{50}$ parent line | | | | | | | | | |
|---|---|---|---|---|---|---|---|---|---|
| Compound | CH1 | $CH1cis^R$ | RF | $CH1dox^R$ | RF | A2780 | $A2780cis^R$ | RF | SKOV3 |
| D | >100 | >100 | - | - | - | 67 | >100 | - | >100 |
| E | 19.5 | 22 | 1.1 | - | - | 31.5 | 56 | | 1.8 |
| O | >100 | >100 | - | 17.5 | - | 40 | >100 | - | >100 |
| J | 92 | 100 | 1 | >100 | - | 41 | 50 | 1.2 | >100 |
| $Y_2$ | 56 | 61 | 1.1 | 40 | 0.7 | 80 | 90 | 1.1 | 47 |
| I | 6.35 | 6.4 | 1 | 0.425 | 0.07 | 14.5 | 14.5 | 1 | 5.6 |
| M | 5.4 | 5.5 | 1 | 1.5 | 0.3 | 44 | 50 | 1.1 | 50 |
| N | 15.5 | 16 | 1 | 5.1 | 0.3 | 25.5 | 94 | 3.7 | 45 |
| P | 7.2 | 8.9 | 1.2 | 1.5 | 0.2 | 27 | 25 | 0.9 | 13.5 |
| Q | 5.0 | 5.8 | 1.2 | 1.05 | 0.2 | 39 | 29.5 | 0.8 | >100 |
| T | 4.6 | 3.7 | 0.8 | 4.2 | 0.9 | 7.7 | 20 | 2.6 | 19 |
| R | 16 | 13.5 | 0.8 | 5.3 | 0.3 | 39 | 89 | 2.3 | 80 |
| S | 65 | >100 | - | 14.5 | 0.2 | 18 | 62 | 3.4 | >100 |
| X | 15.1 | 16.5 | 1.1 | 17 | 1.1 | 25 | 21 | 0.8 | 25 |
| V | 48 | 42 | 0.9 | 40 | 0.8 | 19 | 40 | 2.1 | 98 |
| W | 48 | 46 | 0.9 | 58 | 1.2 | 17 | 10.5 | 0.6 | >100 |

Table 3

| **The 96 hour $IC_{50}$ values (in μM) for the *in vitro* growth inhibition of human ovarian cell lines**<br>Two of the cell lines are resistant to cisplatin and one to doxorubicin. RF is the resistance factor: $IC_{50}$ resistant line/ $IC_{50}$ parent line | | | | | | | | | |
|---|---|---|---|---|---|---|---|---|---|
| Compound | CH1 | $CH1cis^R$ | RF | $CH1dox^R$ | RF | A2780 | $A2780cis^R$ | RF | SKOV3 |
| $I_{14}$ | 15.0 | 17.0 | 1.1 | 7.8 | 0.5 | - | 19 | - | 15,0 |
| $Q_{14}$ | 2.0 | 2.8 | 1.4 | 1.9 | 0.9 | - | 2.3 | - | 4.7 |

## Table 4

## % Inhibition at a Given Concentration

| Compound | *Leishmania donovani* | | | | | *Trypanosoma cruzi* | | | | | *Trypanosoma brucei* | | | | | | | |
|---|---|---|---|---|---|---|---|---|---|---|---|---|---|---|---|---|---|---|
| | 30μM | 10μM | 3μM | 1μM | $ED_{50}$ | 30μM | 10μM | 3μM | 1μM | $ED_{50}$ | 30μM | 10μM | 3μM | 1μM | 0.3μM | 0.1μM | 0.03μM | $ED_{50}$ |
| A | 85.7 | 1.2 | 4.5 | 0.5 | 17.8 | T/100 | T | T | - | - | 100 | 100 | 100 | 100 | 100 | 100 | 83.3 | |
| $A_1$ | 54.2 | 0.2 | 0 | 0 | 28.0 | T | T | T | - | - | 100 | 100 | 100 | 100 | 100 | 100 | 100 | |
| $A_2$ | 45.5 | 27.0 | 0.2 | 0 | 35.9 | T | T | T | 3.8 | - | 100 | 100 | 100 | 100 | 86.4 | 62.7 | 50.0 | |
| $A_3$ | 97.5 | 11.6 | 0 | 0 | 41.2 | 99.8 | 100 | 85.8 | 30.3 | | 100 | 100 | 100 | 100 | 100 | 44.6 | | |
| $A_4$ | 100 | 31.5 | 0 | 0 | 15.0 | 31.3 | 9.3 | 6.0 | 3.0 | | 100 | 100 | 100 | 100 | | | | |
| $A_5$ | 96.9 | 44.9 | 0 | 0 | 12.0 | 58.8 | 43.8 | 6.5 | 2.0 | | 100 | 100 | 100 | 100 | | | | |
| $A_6$ | 96.7 | 30.8 | 2.1 | 0 | 16.0 | 30.8 | 5.0 | 0 | 0 | | 100 | 100 | 100 | 100 | 100 | 75.4 | | |
| $A_7$ | 94.6 | 75.1 | 0 | 0 | 7.7 | 38.0 | 5.0 | 0 | 0 | | 100 | 100 | 100 | 93.0 | 0 | 0 | | |
| $A_8$ | 100 | 82.8 | 3.9 | 0 | 6.8 | 38.0 | 4.0 | 0 | 0 | | 100 | 100 | 100 | 100 | | | | |
| $A_9$ | 98.7 | 61.5 | 0.8 | 0 | 8.2 | | | | | | 100 | 100 | 100 | 100 | 100 | 32.5 | | |
| $A_{10}$ | 99.5 | 92.2 | 0 | 0 | 6.2 | | | | | | 100 | 100 | 100 | 100 | 100 | 81.6 | | |
| $A_{11}$ | T/100 | T/100 | 96.5 | 2.0 | 2.4 | | | | | | 100 | 100 | 100 | 100 | 75.5 | 0 | | |
| $A_{12}$ | 96.1 | 91.7 | 27.5 | 5.0 | 5.2 | 100 | 100 | 100 | 72.3 | | 100 | 100 | 100 | 100 | 98.5 | 83.8 | | |
| $A_{13}$ | 99.1 | 94.9 | 22.0 | 0 | 5.5 | 99.3 | 99.0 | 76.0 | 52.0 | | 100 | 100 | 100 | 100 | 100 | 26.2 | | |

T means toxic to the host cells

# Table 5

## % Inhibition at a Given Concentration

| Compound | *Leishmania donovani* | | | | | *Trypanosoma cruzi* | | | | | *Trypanosoma brucei* | | | | | | |
|---|---|---|---|---|---|---|---|---|---|---|---|---|---|---|---|---|---|
| | 30μM | 10μM | 3μM | 1μM | $ED_{50}$ | 30μM | 10μM | 3μM | 1μM | $ED_{50}$ | 30μM | 10μM | 3μM | 1μM | 0.3μM | 0.1μM | 0.03μM |
| E | 67.1 | 8.0 | 0 | 0 | 19.6 | T | T | 0 | 0 | - | 100 | 100 | 98.4 | 81.9 | 73.0 | 65.4 | 66.7 |
| Y | 100 | 97.0 | 1.1 | 0 | 6.6 | | | | | | 100 | 100 | 100 | 100 | 0 | 0 | |
| I | 93.3 | 89.5 | 47.9 | 2.5 | 3.1 | 100 | 97.0 | 73.5 | 59.7 | | 100 | 100 | 100 | 100 | 83.8 | 99.2 | |
| M | 100 | 92.8 | 5.4 | 0 | 6.8 | T | T | T | T/O | - | 100 | 100 | 100 | 100 | 100 | 96.5 | |
| N | 97.3 | 0 | 0 | 0 | 20.0 | 28.3 | 4.8 | 0 | 0 | | 100 | 100 | 100 | 100 | 100 | 40.1 | |
| P | 99.7 | 93.1 | 96.2 | 96.9 | - | T/0 | 0 | 0 | 0 | - | 100 | 100 | 100 | 100 | 100 | 87.8 | 50.0 |
| Q | T/100 | T/100 | 99.8 | 31.4 | 1.8 | | | | | | 100 | 100 | 100 | 100 | 100 | 0 | |
| O | 0 | 0 | 0 | 0 | - | 11.0 | 2.8 | 3.0 | 1.0 | | 100 | 100 | 100 | 64.7 | 15.2 | 0 | |
| U | 1.5 | 0 | 0 | 0 | - | 18.0 | 0 | 0 | 0 | - | 100 | 100 | 68.4 | 0 | 0 | 0 | |
| T | 97.9 | 96.9 | 0 | 0 | - | T/0 | 0 | 0 | 0 | - | 100 | 100 | 100 | 100 | 78.9 | 54.2 | |
| R | 14.4 | 0 | 0 | 0 | - | 60.1 | 65.8 | 57.7 | 60.8 | | 100 | 100 | 100 | 100 | 29.6 | 4.9 | |
| S | 0 | 0 | 0 | 0 | - | 33.4 | 53.3 | 53.3 | 60.9 | | 100 | 100 | 98.3 | 44.4 | 22.5 | 0 | |
| X | 100 | 100 | 100 | 0 | - | | | | | | 100 | 100 | 100 | 100 | 0 | 0 | |
| V | 0 | 0 | 0 | 0 | - | T/0 | 0 | 0 | 0 | - | 100 | 100 | 100 | 0 | 0 | 0 | |
| W | 0 | 0 | 0 | 0 | - | T/8.3 | 0 | 0 | 0 | - | 100 | 59.8 | 0 | 0 | 0 | 0 | |

T means toxic to macrophages

T/100 means toxic to macrophages, 100% Inhibition

T/0 means toxic to macrophages, 0% inhibition.

## Table 6

### % Inhibition at a Given Concentration

| Compound | *Leishmania donovani* | | | | | *Trypanosoma cruzi* | | | | | *Trypanosoma brucei* | | | | | | |
|---|---|---|---|---|---|---|---|---|---|---|---|---|---|---|---|---|---|
| | 30μM | 10μM | 3μM | 1μM | $ED_{50}$ | 30μM | 10μM | 3μM | 1μM | $ED_{50}$ | 30μM | 10μM | 3μM | 1μM | 0.3μM | 0.1μM | 0.03μM |
| $I_{17}$ | T/100 | T/100 | 99 | 99 | - | | | | | | 100 | 100 | 100 | 100 | 0 | 0 | |
| $Q_{17}$ | T/100 | T/100 | T/100 | 100 | - | | | | | | 100 | 100 | 100 | 100 | 100 | 100 | |

Table 7

Activity of Selected compounds Q, I, G and $A_{11}$ against Chloroquine resistant K1 *P. falciparum in vitro*.

| | % Inhibition (compound duplicates) | | | | | | | | | % Inhibition | | |
|---|---|---|---|---|---|---|---|---|---|---|---|---|
| Concn. (μM) | Q(1) | Q(2) | I(1) | I(2) | G(1) | G(2) | $A_{11}$(1) | $A_{11}$(2) | Concn. (μM) MQ | MQ(1) | MQ(2) | inf-uninf |
| 30 | 99.8 | 99.8 | 93.2 | 90.9 | 98.9 | 98.4 | 99.2 | 99.2 | 2 | 96.3 | 94.7 | 11878.38 |
| 15 | 99.6 | 97.0 | 14.2 | 9.4 | 95.9 | 97.3 | 99.4 | 99.3 | 1 | 94.9 | 91.6 | |
| 7.5 | 97.6 | 98.6 | 1.9 | 2.1 | 93.3 | 95.3 | 98.8 | 98.9 | 0.5 | 96.4 | 95.3 | |
| 3.75 | 77.5 | 60.8 | 0 | 0 | 49.6 | 71.5 | 86.4 | 92.9 | 0.25 | 98.0 | 97.2 | |
| 1.875 | 55.9 | 46.1 | 0 | 0 | 11.4 | 36.0 | 0 | 53.1 | 0.125 | 93.6 | 94.8 | |
| 0.9375 | 6.2 | 0 | 0 | 0 | 17.6 | 44.1 | 0 | 0 | 0.0625 | 77.5 | 56.9 | |
| 0.46875 | 0 | 0 | 0 | 0 | 0 | 17.0 | 0 | 0 | 0.03125 | 0.5 | 0 | |
| 0.234375 | 0 | 0 | 0 | 0 | 0 | 0 | 0 | 0 | 0.015625 | 0 | 0 | |
| $IC_{50}$ | 1.9 | 2.8 | 19.9 | 21.2 | 3.7 | 2.0 | 3.5 | 1.9 | $IC_{50}$MQ | 0.05 | 0.06 | |
| $IC_{50}$ (avg) | 2.4 | | 20.6 | | 2.9 | | 2.7 | | $IC_{50}$ (avg) MQ | 0.06 | | |

**References**

[1] Jennette, K., Lippard, S.J., Vassiiiades, G. and Bauer, W. (1974) *Proc. Natl. Acad.Sci.USA, 71,* 3839-3843.
[2] Bond, P.J., Langridge,R., Jennette,K.W. and Lippard,S.J.(1975) *Proc. Natl. Acad. Sci. USA,* 72, 4825-29.
[3] Howe-Grant, M., Wu,K.C., Bauer,W.R and Lippard,S.J. (1976) *Biochemistry,* 15, 4339-4346.
[4] Peyratout, C.S., Aldridge, T.K., Crites, D.K. and McMillin, D.R. (1995) *Inorg. Chem.*, 34, 4484-89.
[5] Quigley, G.J., Wang, A.H.-J., Ughetto, G., van der Marel, G., van Boom, J.H. and Rich, A. *Proc. Natl. Acad. Sci. USA,* 1980, 77, 7204.
[6]Comess, K.M. and Lippard, S.J. in Molecular Aspects of Anticancer Drug-DNA Interactions Volume 1, ed. by Neidle, S. and Waring, M., The MacMillan Press, 1993, pp. 134-168.
[7] Hollis, L.S. Sundquist, W.I, Burstyn, J.N., Heiger-Bernays, W.J., Bellon, S.F., Ahmed, S.F., Amundsen, A.R., Stern, E.W. and Lippard, S.J., *Cancer Res*. 1991, 51, 1866; Hollis, L.S., Amundsen, A.R. and Stern, E.W., *J. Med. Chem.,* 1989, 32, 128.
[8] du Bois, A., Vach,W., Cramer-Giraud, U., Thomssen, C., Glaubitz, M., Fiola, M., *Anti-CancenDrugs,* 1995, 6, 645-651.
[9] E. C. Constable and M. D. Ward, *J. Chem. Soc. Dalton Trans.,* 1990, 1405-1409.
[10] J. -M. Lehn, J. -P. Sauvage, J. Simon, R. Ziessel, C. Piccinni-Leopardi, G. Germain, J. -P. Declercq and M. Van Meerssche, *Nouveau Journal de chimie,* 1983, 7, 413-420.

**Claims**

**1.** A water soluble 2,2':6',2"-terpyridine Pt (II) complex having a cation of the structure

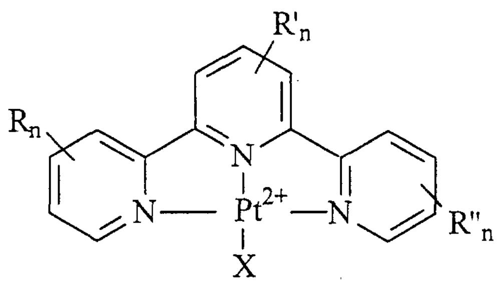

and a counterion,

where X is a halide ion,
R, R' and R" are the same or different and each is H, alkyl, aryl, aralkyl, alkaryl, acyl, halogen, haloalkyl, haloaryl, hydroxyalkyl, hydroxyaryl, aminoalkyl, aminoaryl, primary, secondary or tertiary amino, hydrazino, alkylhydrazino, alkoxy, aralkoxy, nitrile, ester, amido, nitro, azido, or aziridino, or two said groups R, R' and R" together form a divalent chain which is joined to at least one other complex of the above structure so as to form a dimeric species, and n is 1, 2 or 3, for use in the treatment of a tumour.

**2.** A water soluble 2,2':6',2"-terpyridine Pt (II) complex having a cation of the structure

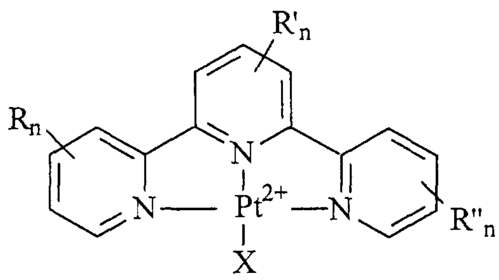

and a counterion,

where X is a halide ion,
R, R' and R" are the same or different and each is H, alkyl, aryl, aralkyl, alkaryl, acyl, halogen, haloalkyl, haloaryl, hydroxyalkyl, hydroxyaryl, aminoalkyl, aminoaryl, primary, secondary or tertiary amino, hydrazino, alkylhydrazino, alkoxy, aralkoxy, nitrile, ester, amido, nitro, azido, or aziridino, or the said groups R, R' and R" together form,a divalent chain which is joined to at least one other complex of the above structure so as to form a dimeric species, and n is 1, 2 or 3, for use in the treatment of a protozoa.

**3.** A complex according to claim 1 or 2, wherein the R, R or R" is a methoxy or halo group.

**4.** A complex according to any one of claims 1 to 3, wherein R' is 4'-(4-substituted)-phenyl.

**5.** A complex according to claim 4, wherein the 4-substituent is methyl or bromo.

**6.** A complex according to any one of claims 1 to 5, wherein R' is amino, e.g. $NH_2$ or NHR or $NR_2$, hydrazino, alkylhydrazino or aziridino or azido.

**7.** A complex according to any one of claims 1 to 6, wherein the counterion is tetrafluoroborate.

**8.** A complex according to any one of the preceding claims wherein X is chlorine.

**9.** A complex according to claim 8 wherein the compound is chloro-(2,2':6',2"-terpyridine) platinum (II) tetrafluoroborate.

**10.** A method of preparing an anti-tumour agent, which method comprises bringing a complex into a form suitable for administration, said complex being as defined in any one of claims 1 to 9.

**11.** A method of preparing an anti-protozoal agent, which method comprises bringing a complex as defined in any one of claims 1 to 9 into a form suitable for administration.

**12.** A preparation suitable for use in the treatment of a tumour which comprises a complex as defined in any one of claims 1 to 9.

**13.** A preparation suitable for use in the treatment of protozoa which comprises a complex as defined in any one of claims 1 to 9.

**14.** A ribonucleoside or 2'-deoxyribonucleoside base-labelled with a 2,2':6',2"-terpyridine Pt (II) complex as defined in any one of claims 1 to 9.

**15.** A ribonucleoside or deoxyribonucleoside as claimed in claim 14 which is G or dG base-labelled at N-7, or A or dA or C or dC base-labelled at N-3 and N-4.

**16.** A base-labelled ribonucleoside or deoxyribonucleoside according to claim 14 or 15 for use in disrupting DNA replication.

Fig.1.

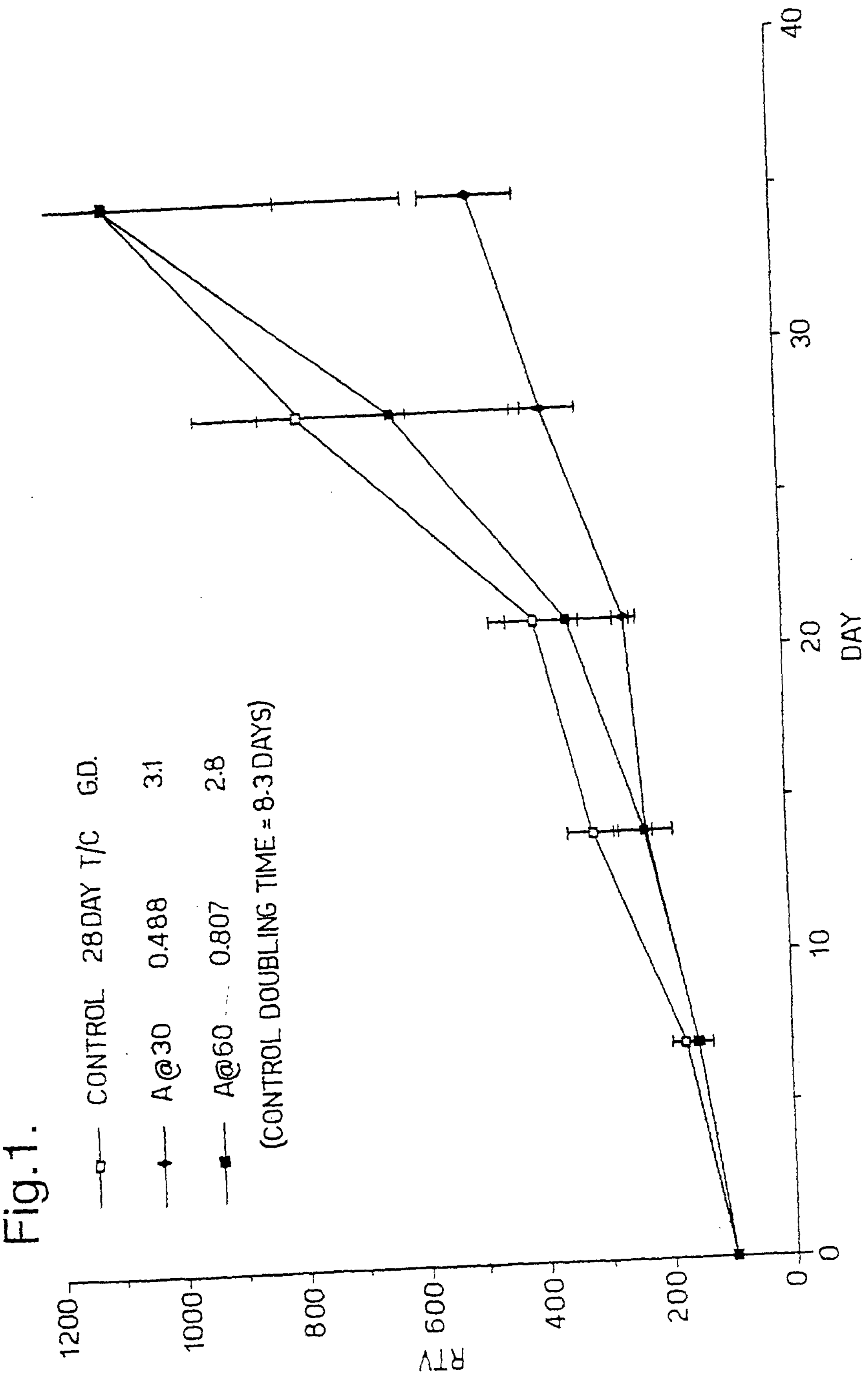
CONTROL 28DAY T/C G.D.
A@30 0.488 3.1
A@60 0.807 2.8
(CONTROL DOUBLING TIME = 8.3 DAYS)
RTV
1200
1000
800
600
400
200
0
DAY
0
10
20
30
40

European Patent Office

# EUROPEAN SEARCH REPORT

Application Number
EP 01 12 1776

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | W. D. MCFADYEN ET AL.: "Activity of Platinum(II) Intercalating Agents against Murine Leukemia L1210" JOURNAL OF MEDICINAL CHEMISTRY, vol. 28, no. 8, 1985, pages 1113-6, XP000670165 * page 1113, abstract: page 1114, chart I, compound 3a; table I, compound 3a * --- | 1,16 | C07F15/00 C07D213/06 C07D213/22 C07H19/067 C07H19/073 C07H19/167 C07H19/173 |
| A,P | A. MCCOUBREY ET AL.: "4-Picoline-2,2':6',2"-terpyridine-platinum(II) - a potent intercalator of DNA" FEBS LETTERS, vol. 380, no. 1,2, 1996, pages 73-8, XP000670178 * page 73, scheme 1; page 74, left-hand column, paragraph 2.3. * --- | 1,7-9 | |
| A,D | K. W. JENNETTE ET AL.: "Metallointercalation Reagents. 2-Hydroxyethanethiolato(2,2',2"-terpyridine)-platinum(II) Monocation Binds Strongly to DNA By Intercalation" PROCEEDINGS OF THE NATIONAL ACADEMY OF SCIENCES, vol. 71, no. 10, 1974, pages 3839-43, XP000197321 * page 3839, figure 1 * --- | 1,16 | TECHNICAL FIELDS SEARCHED (Int.Cl.7) C07F C07D |
| A,D | M. HOWE-GRANT ET AL.: "Binding of Platinum and Palladium Metallointercalation Reagents and Antitumor Drugs to Closed and Open DNAs" BIOCHEMISTRY, vol. 15, 1976, pages 4339-46, XP000670179 * page 4340, figure 1 * --- -/-- | 1,16 | |

The present search report has been drawn up for all claims

| Place of search | Date of completion of the search | Examiner |
|---|---|---|
| BERLIN | 25 October 2001 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 12 1776

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | W. D. MCFADYEN ET AL.: "Binuclear platinum (II)-terpyridine complexes" THE BIOCHEMICAL JOURNAL, vol. 238, no. 3, 1986, pages 757-63, XP000197312 * the whole document * --- | 1,16 | |
| A | W. D. MCFADYEN ET AL.: "The binding of binuclear platinum(II)-terpyridine complexes to DNA" THE BIOCHEMICAL JOURNAL, vol. 242, no. 1, 1987, pages 177-83, XP000197311 * the whole document * --- | 1,16 | |
| A | L. P. G. WAKELIN ET AL.: "Interaction of phenylthiolato-(2,2',2"-terpyridine)-platinum(II) cation with DNA" THE BIOCHEMICAL JOURNAL, vol. 222, no. 1, 1984, pages 203-15, XP000197313 * the whole document * --- | 1,16 | TECHNICAL FIELDS SEARCHED (Int.Cl.7) |
| A | S. L. PINNOW ET AL.: "Selective Labeling of the Enzyme Papain with Chloro(terpyridine)platinum(II)" CROATICA CHEMICA ACTA, vol. 64, no. 3, 1991, pages 519-28, XP000197318 * page 520, left-hand formula * --- -/-- | 1 | |

The present search report has been drawn up for all claims

| Place of search | Date of completion of the search | Examiner |
|---|---|---|
| BERLIN | 25 October 2001 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 01 12 1776

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | G. ANNIBALE ET AL.: "New routes for the synthesis of chloro(diethylenetriamine)-platinum(II)chloride and chloro-(2,2':6',2"-terpyridine)platinum(II) chloride dihydrate" POLYHEDRON, vol. 14, no. 3, 1995, pages 451-3, XP000197341 * page 452, left-hand column, lines 5-13 * --- | 1 | |
| A | B. PITTERI ET AL.: "Nucleophilic Displacement of Halides from Monocationic Platinum(II) Complexes containing Neutral Tridentate Chelating Ligands with Sulfur and Nitrogen Donors: Kinetics and Equilibria" JOURNAL OF THE CHEMICAL SOCIETY, DALTON TRANSACTIONS, no. 23, 1995, pages 3853-9, XP000670168 * page 3853, right-hand column, last paragraph to page 3854, left-hand column, first paragraph; page 3855, table 1, paragraph (d); page 3856, table 2, paragraph "L = terpy" * --- | 1 | TECHNICAL FIELDS SEARCHED (Int.Cl.7) |
| A,D | C .S. PEYRATOUT ET AL.: "DNA-Binding Studies of a Bifunctional Platinum Complex That Is a Luminescent Intercalator" INORGANIC CHEMISTRY, vol. 34, no. 17, 1995, pages 4484-9, XP000197315 * the whole document * ----- | 1 | |

The present search report has been drawn up for all claims

| Place of search | Date of completion of the search | Examiner |
|---|---|---|
| BERLIN | 25 October 2001 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)